# EUROPEAN PATENT APPLICATION

(11) **EP 4 596 713 A1**
(43) Date of publication of application: **06.08.2025**
(21) Application number: 24382113.9
(22) Date of filing: 05.02.2024
(51) Int. Cl.: C12Q 1/6806

(54) **DNA WITH ENHANCED RESISTANCE AGAINST EXONUCLEASES AND METHODS FOR THE PRODUCTION THEREOF**

(71) Applicant: 4basebio UK Ltd, Cambridge CB24 5QE (GB)
(72) Inventor: LANCKRIET, Heikki, Over, Cambridge, CB24 5QE (GB); PICHER, Ángel, E-28049 Cantoblanco, Madrid (ES); WALKER, Amy, Over, Cambridge, CB24 5QE (GB); PAVLICKOVA, Milena, Over, Cambridge, CB24 5QE (GB)
(74) Representative: Boult Wade Tennant LLP

(57) **Abstract**

The present invention relates to methods for producing a protected deoxyribonucleic acid (DNA) product with enhanced resistance to nuclease digestion. The methods comprise digesting a double-stranded DNA molecule with an endonuclease to generate a digested double-stranded DNA molecule comprising a first strand and a second strand, wherein the first strand has a 5' overhang; and extending the second strand 5' to 3' using a polymerase in the presence of one or more nuclease-resistant deoxynucleotide triphosphates to generate the protected DNA product, wherein the protected DNA product comprises an extended second strand comprising at least one nuclease-resistant nucleotide incorporated into the extended second strand by the polymerase using the 5' overhang of the first strand as template. Also provided are protected DNA products and uses thereof.

## Description

### TECHNICAL FIELD

The present invention relates to methods for producing a protected deoxyribonucleic acid (DNA) product with enhanced resistance to nuclease digestion. Also provided are protected DNA products and uses thereof.

### BACKGROUND

DNA is susceptible to degradation by nucleases which are naturally occurring enzymes within organisms and which have a vital role in the regulation of many cellular processes, while also protecting against foreign DNA species. Enzymatic DNA degradation can render gene therapies ineffective and is a substantial consideration when developing gene therapies or DNA vaccines.

Considerable efforts have been made to extend the effective molecular lifetime of nucleic acids by increasing resistance of the nucleic acid molecules to both extracellular and intracellular nucleases.

For linear molecules, one of the proposed solutions includes the use of phosphorothioated nucleotides (i.e. 2'-deoxynucleotides-5'-(α-thio)-triphosphate).

Phosphorothioated nucleotides comprise a sulphur atom instead of a non-bridging oxygen atom. These modified nucleotides show comparable physical and chemical characteristics to corresponding unmodified nucleotides, but are resistant to exonuclease digestion. As such, the incorporation of the phosphorothioate functional group can prolong the half-life of the nucleic acid molecule.

Phosphorothioate modifications are used in nucleic acid drug development programmes. In therapeutic nucleic acids, the phosphorothioated nucleotides are incorporated into short, single-stranded polynucleotide chains. For example, an antisense oligonucleotide fomivirsen is a 21-mer phosphorothioate oligodeoxynucleotide used to treat cytomegalovirus retinitis (Stein and Castanotto, "FDA-approved oligonucleotide therapies in 2017." Molecular Therapy 25.5 (2017): 1069-1075). Similarly, pegaptanib (brand name Macugen) is a short (27-nucleotides) aptamer with a phosphorothioate 3'-3' deoxythymidine cap used for treating age-related macular degeneration of the retina.

Phosphorothioate modifications have also been used to increase resistance of a DNA fragment to exonuclease digestion (Putney et al. "A DNA fragment with an alpha-phosphorothioate nucleotide at one end is asymmetrically blocked from digestion by exonuclease III and can be replicated in vivo." Proceedings of the National Academy of Sciences 78.12 (1981): 7350-7354).

WO2023/006978 A1 discloses methods for producing a deoxyribonucleic acid (DNA) product with enhanced resistance to nuclease digestion based on ligating adaptor molecules to a double-stranded DNA molecule.

Resistance to nuclease digestion can also be accomplished by using closed DNA molecules, such as plasmids or minicircles. However, plasmids and minicircles have limited utility *in vivo* due to their frequent contamination with toxic agents derived from cell components, fidelity issues that alter the sequence of interest, and presence of different species (supercoiled, linear and open circular).

Alternatively, resistance to nuclease digestion may be accomplished by producing closed linear DNA molecules. For example, WO2010/086626 A1 describes a method for producing a closed linear DNA by utilizing a protelomerase. However, this method is limited in that the action of protelomerase produces the same sequence at both ends of the closed linear DNA molecule.

Thus, a need exists for a more flexible method for producing a protected DNA product with an enhanced resistance to nuclease (e.g. exonuclease) digestion.

### DESCRIPTION

### 1. Methods for producing a protected DNA product

The invention provides a method for producing a protected deoxyribonucleic acid (DNA) product, wherein the method comprises: digesting a double-stranded DNA molecule with an endonuclease to generate a digested double-stranded DNA molecule comprising a first strand and a second strand, wherein the first strand has a 5' overhang; and extending the second strand 5' to 3' using a polymerase in the presence of one or more nuclease-resistant deoxynucleotide triphosphates to generate the protected DNA product, wherein the protected DNA product comprises an extended second strand comprising at least one (preferably at least two) nuclease-resistant nucleotide(s) incorporated into the extended second strand by the polymerase using the 5' overhang of the first strand as template.

The invention provides a method for producing a protected deoxyribonucleic acid (DNA) product, wherein the method comprises: digesting a double-stranded DNA molecule with an endonuclease to generate a digested double-stranded DNA molecule comprising a first strand and a second strand, wherein the first strand has a 5' overhang and the second strand has a 5' overhang; and extending the first strand and the second strand 5' to 3' using a polymerase in the presence of one or more nuclease-resistant deoxynucleotide triphosphates to generate the protected DNA product, wherein the protected DNA product comprises (i) an extended first strand comprising at least one (preferably at least two) nuclease-resistant nucleotide(s) incorporated into the extended first strand by the polymerase using the 5' overhang of the second strand as template, and (ii) an extended second strand comprising at least one (preferably at least two) nuclease-resistant nucleotide(s) incorporated into the extended second strand by the polymerase using the 5' overhang of the first strand as template.

The invention provides a method for producing a protected deoxyribonucleic acid (DNA) product, wherein the method comprises:
(a) amplifying a DNA template molecule to generate a double-stranded DNA molecule;
(b) digesting the double-stranded DNA molecule with an endonuclease to generate a digested double-stranded DNA molecule comprising a first strand and a second strand, wherein the first strand has a 5' overhang; and
(c) extending the second strand 5' to 3' using a polymerase in the presence of one or more nuclease-resistant deoxynucleotide triphosphates to generate the protected DNA product, wherein the protected DNA product comprises an extended second strand comprising at least one (preferably at least two) nuclease-resistant nucleotide(s) incorporated into the extended second strand by the polymerase using the 5' overhang of the first strand as template.

The invention provides a method for producing a protected deoxyribonucleic acid (DNA) product, wherein the method comprises:
(a) amplifying a DNA template molecule to generate a double-stranded DNA molecule, wherein the amplifying comprises rolling circle amplification;
(b) digesting the double-stranded DNA molecule with an endonuclease to generate a digested double-stranded DNA molecule comprising a first strand and a second strand, wherein the first strand has a 5' overhang; and
(c) extending the second strand 5' to 3' using a polymerase in the presence of one or more nuclease-resistant deoxynucleotide triphosphates to generate the protected DNA product, wherein the protected DNA product comprises an extended second strand comprising at least one (preferably at least two) nuclease-resistant nucleotide(s) incorporated into the extended second strand by the polymerase using the 5' overhang of the first strand as template.

In step (b) (i.e. the step of digesting the double-stranded DNA molecule), the first strand may have a 5' overhang and the second strand may have a 5' overhang and wherein step (c) (i.e. the step of extending) may comprise:
extending the first strand and the second strand 5' to 3' using a polymerase in the presence of one or
more nuclease-resistant deoxynucleotide triphosphates to generate the protected DNA product,
wherein the protected DNA product may comprise (i) an extended first strand comprising at least one (preferably at least two) nuclease-resistant nucleotide(s) incorporated into the extended first strand by the polymerase using the 5' overhang of the second strand as template, and (ii) an extended second strand comprising at least one (preferably at least two) nuclease-resistant nucleotide(s) incorporated into the extended second strand by the polymerase using the 5' overhang of the first strand as template.

Thus, the invention provides a method for producing a protected deoxyribonucleic acid (DNA) product, wherein the method comprises:
(a) amplifying a DNA template molecule to generate a double-stranded DNA molecule, wherein the amplifying comprises rolling circle amplification
(a) digesting the double-stranded DNA molecule with an endonuclease to generate a digested double-stranded DNA molecule comprising a first strand and a second strand, wherein the first strand has a 5' overhang and the second strand has a 5' overhang; and
(b) extending the first strand and the second strand 5' to 3' using a polymerase in the presence of one or more nuclease-resistant deoxynucleotide triphosphates to generate the protected DNA product, wherein the protected DNA product comprises (i) an extended first strand comprising at least one (preferably at least two) nuclease-resistant nucleotide(s) incorporated into the extended first strand by the polymerase using the 5' overhang of the second strand as template, and (ii) an extended second strand comprising at least one (preferably at least two) nuclease-resistant nucleotide(s) incorporated into the extended second strand by the polymerase using the 5' overhang of the first strand as template.

It is known that DNA polymerases synthesise DNA in the 5' to 3' direction, adding a dNTP to the 3' hydroxyl group of a growing strand. Thus, in the present invention the polymerase extends the first and/or second strands in the 5' to 3' direction starting from the 3' end of the first and/or second strand.

The 5' overhang of the first strand may have the same length or a different length to the 5' overhang of the second strand. The 5' overhang of the first strand may be the same length as the 5' overhang of the second strand. The 5' overhang may be 1-100 nucleotides in length. The 5' overhang may be 1-75 nucleotides in length. The 5' overhang may be 1-50 nucleotides in length. The 5' overhang may be 1-25 nucleotides in length. The 5' overhang may be 1-10 nucleotides in length. The 5' overhang may be at least 1, 2, 3 or 4 nucleotides in length. Preferably, the 5' overhang is at least 4 nucleotides in length (such as 4 nucleotides in length). Preferably, the 5' overhang is no more than 10 nucleotides in length.

The 5' overhang of the first strand may have the same sequence or a different sequence to the 5' overhang of the second strand. Preferably, the 5' overhang of the first strand has the same sequence as the 5' overhang of the second strand.

The 5' overhang may consist of one, two, three or four types of nucleotide selected from cytosine, guanine, thymine and/or adenine. Thus, the 5' overhang may consist of only one type of nucleotide (e.g. all cytosine), two types of nucleotide (e.g. a combination of cytosine and guanine), three types of nucleotide (e.g. a combination of cytosine, guanine and thymine) or four types of nucleotide (e.g. a combination of cytosine, guanine, thymine or adenine) selected from cytosine, guanine, thymine and/or adenine. Preferably, the 5' overhang consists of no more than three types of nucleotide selected from cytosine, guanine, thymine and/or adenine. More preferably, the 5' overhang consists of no more than two types of nucleotide selected from cytosine, guanine, thymine and/or adenine.

The nuclease-resistant deoxynucleotide triphosphates may consist of one, two, three or four types of nuclease-resistant deoxynucleotide triphosphate selected from guanine triphosphate, cytosine triphosphate, adenine triphosphate and/or thymine triphosphate. Thus, the nuclease-resistant deoxynucleotide triphosphate may consist of only one type of nuclease-resistant deoxynucleotide triphosphate (e.g. all cytosine triphosphate), two types of nuclease-resistant deoxynucleotide triphosphate (e.g. a combination of cytosine triphosphate and guanine triphosphate), three types of nuclease-resistant deoxynucleotide triphosphate (e.g. a combination of cytosine triphosphate, guanine triphosphate and thymine triphosphate) or four types of nuclease-resistant deoxynucleotide triphosphate (e.g. a combination of cytosine triphosphate, guanine triphosphate, thymine triphosphate or adenine triphosphate) selected from cytosine triphosphate, guanine triphosphate, thymine triphosphate and/or adenine triphosphate. Preferably, the nuclease-resistant deoxynucleotide triphosphates consist of no more than three types of nuclease-resistant deoxynucleotide triphosphate selected from cytosine triphosphate, guanine triphosphate, thymine triphosphate and/or adenine triphosphate. More preferably, the nuclease-resistant deoxynucleotide triphosphates consist of no more than two types of nuclease-resistant deoxynucleotide triphosphate selected from cytosine triphosphate, guanine triphosphate, thymine triphosphate and/or adenine triphosphate.

The nuclease-resistant deoxynucleotide triphosphates may be selected to be complementary to the sequence of the 5' overhang. Thus, if the 5' overhang consists of one type of nucleotide, the nuclease-resistant deoxynucleotide triphosphates may consist of the complementary nuclease-resistant deoxynucleotide triphosphates. For example, if the 5' overhang consists of one or more cytosine nucleotides, the nuclease-resistant deoxynucleotide triphosphates may consist of guanine triphosphates. In another example, if the 5' overhang consists of one or more cytosine and thymine nucleotides, the nuclease-resistant deoxynucleotide triphosphates may consist of guanine triphosphates and adenine triphosphates.

The endonuclease may be any endonuclease that generates a 5' overhang (preferably a 5' overhang that is at least two nucleotides in length). The endonuclease may be a restriction endonuclease (such as a Type II restriction endonuclease), RNA-guided DNA endonuclease, nicking endonuclease, meganuclease or homing endonuclease. The Type II restriction endonuclease may be a Type IIP restriction endonuclease (e.g. EcoRI, Hindlll, BamHI or Notl) or a Type IIS restriction endonuclease (e.g. Bsal, Bbvl, Fokl or Sapl).

Type IIS restriction endonucleases cleave the double-stranded DNA molecule outside of the recognition sequence (i.e. an endonuclease target sequence), which means that the recognition sequence (i.e. endonuclease target sequence) is not included in the protected DNA product.

The endonuclease may be an RNA-guided DNA endonuclease. The use of an RNA-guided DNA endonuclease allows any sequence to be cleaved, since a guide RNA (gRNA) can be designed to target any sequence. Alternatively, a sequence of interest, or cassette may be cloned into a universal vector, such that the same gRNA can be used for the digestion to produce protected DNA products.

The RNA-guided DNA endonuclease generates a 5' overhang. The RNA-guided DNA endonuclease may be an endonuclease of Class 2 e.g. Class 2 Type V. The RNA-guided DNA endonuclease may be Cas12a. The RNA-guided DNA endonuclease may be Cpf1 or Mad7. Preferably, the RNA-guided DNA endonuclease is Cpf1.

In the methods in which the endonuclease is an RNA-guided DNA endonuclease, the double-stranded DNA molecule is contacted with the RNA-guided DNA endonuclease and at least one gRNA. The RNA-guided DNA endonuclease and the at least one gRNA may be provided as a ribonucleoprotein (RNP) complex or they be provided separately and then form a RNP complex.

In the case of a nicking endonuclease, the nicking endonuclease may nick the second strand (and/or the first strand). Depending on the melt temperature of the nicked fragment of the second strand with the first strand a 5' overhang of the first strand may spontaneously form. Alternatively, the polymerase extending the second strand may displace the nicked fragment of the second strand. An equivalent process may take place when the nicking endonuclease nicks the first strand.

The homing endonuclease may be I-CeuI, I-SceI, PI-PspI or PI-SceI.

Steps (b) and (c) may be performed in a single contiguous volume. Thus, the step of digesting the double-stranded DNA molecule and the step of extending the first and/or second strand 5' to 3' using a polymerase may be performed in a single contiguous volume.

Preferably, the step of digesting the double-stranded DNA molecule is performed by contacting the double-stranded DNA molecule with the endonuclease in the presence of the polymerase and the one or more nuclease-resistant deoxynucleotide triphosphates.

The invention provides a method for producing a protected deoxyribonucleic acid (DNA) product, wherein the method comprises:
contacting a double-stranded DNA molecule with an endonuclease, a polymerase and one or more nuclease-resistant deoxynucleotide triphosphates to form a single contiguous aqueous volume; and
incubating the single contiguous aqueous volume to generate the protected DNA product, wherein the endonuclease digests the double-stranded DNA molecule to generate a digested double-stranded DNA molecule comprising a first strand and a second strand, wherein the first strand has a 5' overhang; and wherein the polymerase extends the second strand 5' to 3' to generate the protected DNA product, wherein the protected DNA product comprises an extended second strand comprising at least one (preferably at least two) nuclease-resistant nucleotide(s) incorporated into the extended second strand by the polymerase using the 5' overhang of the first strand as template.

The invention provides a method for producing a protected deoxyribonucleic acid (DNA) product, wherein the method comprises:
contacting a double-stranded DNA molecule with an endonuclease, a polymerase and one or more nuclease-resistant deoxynucleotide triphosphates to form a single contiguous aqueous volume; and
incubating the single contiguous aqueous volume to generate the protected DNA product, wherein the endonuclease digests the double-stranded DNA molecule to generate a digested double-stranded DNA molecule comprising a first strand and a second strand, wherein the first strand has a 5' overhang and the second strand has a 5' overhang; and wherein the polymerase extends the first and second strand 5' to 3' to generate the protected DNA product, wherein the protected DNA product comprises (i) an extended first strand comprising at least one (preferably at least two) nuclease-resistant nucleotide(s) incorporated into the extended first strand by the polymerase using the 5' overhang of the second strand as template, and (ii) an extended second strand comprising at least one (preferably at least two) nuclease-resistant nucleotide(s) incorporated into the extended second strand by the polymerase using the 5' overhang of the first strand as template.

The invention provides a method for producing a protected deoxyribonucleic acid (DNA) product, wherein the method comprises:
amplifying a DNA template molecule to generate a double-stranded DNA molecule, wherein the amplifying preferably comprises rolling circle amplification;
contacting the double-stranded DNA molecule with an endonuclease, a polymerase and one or more nuclease-resistant deoxynucleotide triphosphates to form a single contiguous aqueous volume; and
incubating the single contiguous aqueous volume to generate the protected DNA product, wherein the endonuclease digests the double-stranded DNA molecule to generate a digested double-stranded DNA molecule comprising a first strand and a second strand, wherein the first strand has a 5' overhang; and wherein the polymerase extends the second strand 5' to 3' to generate the protected DNA product, wherein the protected DNA product comprises an extended second strand comprising at least one (preferably at least two) nuclease-resistant nucleotide(s) incorporated into the extended second strand by the polymerase using the 5' overhang of the first strand as template.

The invention provides a method for producing a protected deoxyribonucleic acid (DNA) product, wherein the method comprises:
amplifying a DNA template molecule to generate a double-stranded DNA molecule, wherein the amplifying preferably comprises rolling circle amplification;
contacting the double-stranded DNA molecule with an endonuclease, a polymerase and one or more nuclease-resistant deoxynucleotide triphosphates to form a single contiguous aqueous volume; and
incubating the single contiguous aqueous volume to generate the protected DNA product, wherein the endonuclease digests the double-stranded DNA molecule to generate a digested double-stranded DNA molecule comprising a first strand and a second strand, wherein the first strand has a 5' overhang and the second strand has a 5' overhang; and wherein the polymerase extends the first and second strand 5' to 3' to generate the protected DNA product, wherein the protected DNA product comprises (i) an extended first strand comprising at least one (preferably at least two) nuclease-resistant nucleotide(s) incorporated into the extended first strand by the polymerase using the 5' overhang of the second strand as template, and (ii) an extended second strand comprising at least one (preferably at least two) nuclease-resistant nucleotide(s) incorporated into the extended second strand by the polymerase using the 5' overhang of the first strand as template.

The nuclease-resistant deoxynucleotide triphosphates and the nuclease-resistant nucleotides may be phosphorothioated. The nuclease-resistant deoxynucleotide triphosphates may be present in excess concentration. The nuclease-resistant deoxynucleotide triphosphates may be present in a concentration of at least 1 µM, at least 10 µM, at least 100 µM or at least 1 mM. Preferably, the nuclease-resistant deoxynucleotide triphosphates are present at a concentration of at least 10 µM, more preferably at least at least 100 µM.

The polymerase used to extend the first and/or second strand may lack exonuclease activity and/or strand displacement activity. The polymerase may lack 3' to 5' exonuclease activity. The polymerase may lack 5' to 3' exonuclease activity. The polymerase may lack both 3' to 5' exonuclease activity and 5' to 3' exonuclease activity. The polymerase used to extend the first and/or second strand may have exonuclease activity and/or strand displacement activity. The polymerase may have 3' to 5' exonuclease activity. The polymerase may have 5' to 3' exonuclease activity. The polymerase may have both 3' to 5' exonuclease activity and 5' to 3' exonuclease activity. The polymerase used to extend the first and/or second strand may be thermophilic. Thus, the polymerase may retain polymerase activity at temperatures of at least 65 °C, 75 °C, 85 °C and 95 °C. Preferably, the polymerase retains polymerase activity at a temperature of at least 65 °C.

The polymerase used to extend the first and/or second strand may be Taq DNA polymerase, Pfu DNA polymerase, Bst DNA polymerase, Bsu DNA polymerase, Phi29 DNA polymerase, T7 DNA polymerase, Sulfolobus DNA Polymerase IV, 9°N DNA polymerase (Therminator) DNA Polymerase, DNA polymerase I, T4 DNA polymerase, Vent DNA polymerase, or a functional fragment or variant thereof. By "functional" it is meant that the fragments and variants (i.e. polymerase comprising one or more mutations) are able to extend the first and/or second strands. Preferably, the polymerase is Phi29 DNA polymerase such as Phi29 DNA polymerase lacking exonuclease activity (for example Phi29 DNA polymerase D12A/D66A).

The amplification in step (a) (i.e. amplifying a DNA template molecule) may be performed in the absence of nuclease-resistant deoxynucleotide triphosphates. Thus, the double-stranded DNA molecule may be free from nuclease-resistant nucleotides.

The extension in step (c) (i.e. extending the first and/or second strands) may be performed in the absence of non-nuclease-resistant deoxynucleotide triphosphates. Thus, the extended first and/or second strands (i.e. the extended portions of the first and/or second strands) may consist of nuclease-resistant nucleotides.

The method may be performed without a purification step after the amplification in step (a) and before steps (b) and (c). Thus, no purification step may be performed after amplification and before digestion and extension. Purification may comprise precipitation and/or size exclusion chromatography (such as gel filtration).

The extended first strand (i.e. the extended portion of the first strand) may comprise at least two nuclease-resistant nucleotides incorporated into the extended first strand by the polymerase using the 5' overhang of the second strand as template, and/or the extended second strand (i.e. the extended portion of the second strand) may comprise at least two nuclease-resistant nucleotides incorporated into the extended second strand by the polymerase using the 5' overhang of the first strand as template.

Amplification (or step of amplifying) described herein may be an *in vitro* or *in vivo* amplification. Preferably, the amplification is an *in vitro* amplification. For example, the amplification may be performed by rolling circle amplification (RCA), Multiple Annealing and Looping Based Amplification Cycles (MALBAC) method, traditional polymerase chain reaction (PCR), nucleic acid sequence-based amplification (NASBA), loop-mediated isothermal amplification (LAMP), helicase-dependent amplification (HDA), multiple displacement amplification (MDA) and recombinase polymerase amplification (RPA). Preferably, the amplification is rolling circle amplification.

The method may further comprise (after the step of amplifying and before the step of digesting the double-stranded DNA molecule with an endonuclease) a step of heat-deactivation.

The step of heat-deactivation may be performed under conditions sufficient to inactivate the reagents used during the amplification reaction. The step of heat-deactivation may be performed at a temperature of at least 50°C, at least 55°C, at least 60°C, at least 65°C, at least 70°C, at least 75°C, at least 80°C, at least 85°C, at least 90°C, at least 95°C, or at least 100°C. The step of heat-deactivation may be performed for at least 1 min, at least 3 mins, at least 5 mins, at least 10 mins, at least 15 mins, or at least 20 mins. The step of heat-deactivation may be performed at a temperature of 65°C for 10 mins. The amplification (e.g. rolling circle amplification) may be performed at 30°C for at least 20 hours followed by at least 10 mins at 65°C.

The inventors of the present application have surprisingly discovered that large concatemeric products of the rolling circle amplification reaction can be used to produce the protected DNA products described herein. This is surprising as the product of the rolling circle amplification has high viscosity and typically has to undergo purification steps before it can be utilized for downstream applications.

In the method described herein, after the amplification step, the step of digesting the double-stranded DNA molecule with an endonuclease and the step of extending the digested double-stranded DNA molecule (optionally in a single contiguous aqueous volume) may be performed without purifying the product of the amplification reaction. That is to say that the step of step of digesting the double-stranded DNA molecule with an endonuclease and the step of extending the digested double-stranded DNA molecule may be performed directly after the step of amplification. The step of digesting the double-stranded DNA molecule with an endonuclease and the step of extending the digested double-stranded DNA molecule may be performed directly after a step of heat-deactivation.

The inventors of the present application have discovered a method which requires very few steps to produce the protected DNA product described herein. The methods described herein are very time efficient. This is, in part, due to the fact that a step of purification is not required after the amplification reaction. Optionally, the product of the amplification reaction may be heat-deactivated. Surprisingly, when the step of digesting the double-stranded DNA molecule with an endonuclease and the step of extending the digested double-stranded DNA molecule is performed directly after the step of amplification (i.e. without a purification step) comparable yields of the protected DNA product are obtained when compared to a method in which the two steps are separated by purification of the amplification product.

The method may further comprise, after the step of extending the digested double-stranded DNA molecule, a step of purification of the protected DNA product. Purification may comprise precipitation and/or size exclusion chromatography (such as gel filtration).

The method may further comprise, after the step of extending the digested double-stranded DNA molecule, a step of nuclease digestion. The nuclease digestion may be exonuclease digestion, such as exonuclease I and/or exonuclease III digestion. The step of nuclease digestion may take place before or after the step of purification. The step of nuclease digestion may allow for removal of any double-stranded DNA molecules or digested double-stranded DNA molecules which have not been used to produce protected DNA products.

The step of nuclease digestion may be performed after extending the first and/or second strands by contact with an exonuclease (e.g. exonuclease I and/or exonuclease III). The step of nuclease digestion may be performed at a temperature of 5-65 °C, 10-60 °C, 20-50 °C or 30-45 °C. Preferably, the step of nuclease digestion is performed at a temperature of 30-45 °C. The step of nuclease digestion may be performed for at least 10 mins, at least 30 mins, at least 60 mins, or at least 2 hours. Preferably the step of nuclease digestion is performed for at least 2 hours.

The present inventors have surprisingly discovered methods for producing a protected DNA product with enhanced resistance to nuclease digestion. Specifically, the protected DNA product produced by the methods described herein has enhanced resistance to exonuclease digestion (e.g. exonuclease III digestion). The enhanced resistance to exonuclease digestion extends the life of the protected DNA product in a cell (i.e. the protected DNA product has enhanced resistance to intracellular exonucleases) and in a cell-free system (i.e. the protected DNA product has enhanced resistance to extracellular exonucleases). The present inventors have developed a method which relies on using a polymerase to extend a digested double-stranded DNA molecule in the presence of one or more nuclease-resistant deoxynucleotide triphosphates.

The present inventors have discovered that the methods described herein allow for efficient introduction of nuclease-resistant (i.e. protected) nucleotides. The methods of the invention provide DNA products that are protected from digestion by exonucleases that cleave the 3'-end nucleotides (e.g. exonuclease III). Thus, the protected DNA product produced by the methods of the invention has prolonged *in vivo* expression.

As used herein the term "protected nucleotide" or "nuclease-resistant nucleotide" is intended to encompass any type of molecule that provides or enhances resistance to nuclease digestion (especially exonuclease digestion). Although phosphorothioated nucleotides are described herein, the skilled person would appreciate that any molecules that provide resistance to nuclease digestion (e.g. exonuclease III digestion) may be used. For example, other nuclease resistant nucleotides i.e. modified nucleotides that provide or increase resistance to nucleases (e.g. exonucleases) such as 2'-O-methyl nucleotides or 2'-O-methoxyethyl (MOE) nucleotides.

As used herein, the term "phosphorothioated nucleotide" refers to a nucleotide that has an altered phosphate backbone, wherein, the sugar moieties are linked by a phosphorothioate bond. In the phosphate backbone of an oligonucleotide sequence, the phosphorothioate bond contains a sulphur atom as a substitute for a non-bridging oxygen atom. This modification renders the internucleotide linkage resistant to nuclease degradation.

The protected DNA product produced by the methods of the present invention has additional advantageous properties, such as a substantial lack of a bacterial backbone and/or antibiotic resistant genes. Lack of these features is particularly beneficial in the production of a cell delivery system, such as a viral vector or a nanoparticle, for example, for cell therapy. Lack of these features makes the protected DNA product produced by the methods of the present invention particularly suitable for use in a pharmaceutical composition.

Unexpectedly, the present inventors have discovered a method for production of a protected DNA product of enhanced resistance to nuclease digestion which allows for efficient production of large quantities of the protected DNA product with enhanced resistance to exonuclease digestion. The large-scale manufacture of the product may be in a cell-free system, which results in the production of a pure sample comprising the protected DNA product substantively free of bacterial contaminants (e.g. remaining after cell lysis).

A protected DNA product has particular utility as a therapeutic agent (i.e. DNA therapeutic) which can be used to express a gene product in vivo. This is because its protected structure prevents attack by enzymes such as exonucleases, leading to enhanced stability and longevity of gene expression as compared to "unprotected" DNA molecules. Unprotected double-stranded cassettes have been demonstrated to be inefficient with respect to gene expression when introduced into host tissue. This has been attributed to cassette instability due to the action of exonucleases in the extracellular space.

The methods of the invention may be used for production of DNA for *in vitro* expression in a host cell, for example, in DNA vaccines. DNA vaccines typically encode a modified form of an infectious organism's DNA. DNA vaccines are administered to a subject where they then express the selected protein of the infectious organism, initiating an immune response against that protein which is typically protective. DNA vaccines may also encode a tumour antigen in a cancer immunotherapy approach.

The method may produce other types of therapeutic DNA molecules e.g. those used in gene therapy. For example, such DNA molecules can be used to express a functional gene where a subject has a genetic disorder caused by a dysfunctional version of that gene. Examples of such diseases include sickle cell anaemia, cystic fibrosis, Huntington disease, Duchenne's Muscular Dystrophy, Haemophilia A, α1-antitrypsin deficiency, primary ciliary dyskinesia, or respiratory distress syndrome of prematurity. Other diseases where gene therapy may be useful include metabolic diseases, respiratory diseases, inflammatory diseases, autoimmune, chronic and infectious diseases, including such disorders as AIDS, cancer, neurological diseases, cardiovascular disease, hypercholesterolemia, various blood disorders including various anaemias, thalassemia and haemophilia, and emphysema. For the treatment of solid tumours, genes encoding toxic peptides (i.e., chemotherapeutic agents such as ricin, diptheria toxin and cobra venom factor), tumour suppressor genes such as p53, genes coding for mRNA sequences which are antisense to transforming oncogenes, antineoplastic peptides such as tumor necrosis factor (TNF) and other cytokines, or transdominant negative mutants of transforming oncogenes, may be expressed.

The step of incubating a single contiguous aqueous volume to generate the protected DNA product may comprise generating the digested double-stranded DNA molecule by digesting the double-stranded DNA molecule with the endonuclease.

The step of incubating the single contiguous aqueous volume may be performed under conditions that promote endonuclease digestion of the double-stranded DNA molecule and extension of the digested double-stranded DNA molecule by the polymerase to produce the protected DNA product.

The step of incubating the single contiguous aqueous volume may be performed under conditions that promote digestion of the double-stranded DNA molecule and extension of the digested double-stranded DNA molecule.

The digestion and extension steps may lead to at least 5%, at least 10%, at least 15, at least 20%, at least 25%, at least 30%, at least 35%, at least 40, at least 45%, at least 50%, at least 55%, at least 60, at least 65, at least 70%, at least 75, at least 80%, at least 82%, at least 85%, at least 90%, or at least 95% efficient. For example, at least 5%, at least 10%, at least 15, at least 20%, at least 25%, at least 30%, at least 35%, at least 40, at least 45%, at least 50%, at least 55%, at least 60, at least 65, at least 70%, at least 75, at least 80%, at least 82%, at least 85%, at least 90%, or at least 95% of the double-stranded DNA molecules being converted into protected DNA products. Preferably, at least 15% of the double-stranded DNA molecules are converted into protected DNA products.

The double-stranded DNA molecule (e.g. generated by the rolling circle amplification) may first be quantified so that the amount of the double-stranded DNA molecule used as the starting material during the digestion/extension reaction is known. After all the enzymatic reactions, the protected DNA product may be quantified to calculate the efficiency as per the equation above.

DNA quantification methods are known to a person skilled in the art. For example, DNA quantifications may be carried out using the Qubit dsDNA BR assay from ThermoFisher (https://www.thermofisher.com/order/catalog/product/Q32850#/Q32850).

The step of incubating the single contiguous aqueous volume may be performed isothermally. The step of incubating the single contiguous aqueous volume may comprise incubating at a constant temperature. The constant temperature promotes simultaneous digestion of the double-stranded DNA molecule and extension with the polymerase. For example, the constant temperature may be 20°C, 21°C, 22°C, 23°C, 24°C, 25°C, 26°C, 27°C, 28°C, 29°C, 30°C, 31°C, 32°C, 33°C, 34°C, 35°C, 36°C, 37°C, 38°C, 39°C, or 40°C. Preferably, the constant temperature is 30°C. The constant temperature is intended to mean that the temperature does not significantly change during the reaction. The constant temperature is intended to mean that the temperature variation during the step of incubating the single contiguous aqueous volume is less than 10°C, less than 9°C, less than 8°C, less than 7°C, less than 6°C, less than 5°C, less than 4°C, less than 3°C, less than 2°C, or less than 1°C. In a preferred embodiment the temperature during the step of incubating the single contiguous aqueous does not deviate by more than 5°C, preferably by not more than 3°C, even more preferably not more than 1°C. Thus, the constant temperature may be a temperature in a range of 20°C-30°C, 22°C-32°C, 24°C-34°C, 26°C-36°C, 28°C-38°C, 30°C-40°C, 22°C-28°C, 32°C-38°C, 25°C-35°C, 26°C-34°C, 27°C- 33°C, 27.5°C-32.5°C, 28°C-32°C, 28.5°C-31.5°C, 29°C-31°C, or 29.5°C-30.5°C. Preferably, the constant temperature is a temperature in a range of 27.5°C-32.5°C. Alternatively, the constant temperature may be a temperature in a range of 32°C-42°C, 33°C-41°C, 34°C-40°C, 35°C-39°C, 36°C-38°C. Preferably, the constant temperature is a temperature in a range of 34.5°C-39.5°C.

The step of incubating the single contiguous aqueous volume may be performed at a temperature of 10-50°C, 15-45°C, 20-40°C or 25-35°C. The step of incubating the single contiguous aqueous volume may be performed for at least 10, at least 20, at least 30, at least 40, at least 50, or at least 60 min, or at least 2 hours, or at least 4 hours, or at least 8 hours, or at least 12 hours, or at least 16 hours, or at least 20 hours.

The step of incubating the single contiguous aqueous volume may be performed at two different temperatures. For example, the step of incubating the single contiguous aqueous volume may be performed at 15-40°C for at least 10 minutes followed by a temperature of 60-90°C for at least 10 min. The higher temperature typically inactivates the endonuclease. Thus, the method further provides a step of inactivating the endonuclease. The step of incubating the single contiguous aqueous volume may be performed at 30°C for 20 hours and 65°C for 10 min. Preferably, the step of inactivating the endonuclease is performed at a temperature of 70-80°C. The step of inactivating the endonuclease may be performed for at least 1, at least 5, at least 10, at least 20 or at least 30 minutes. Preferably, the step of inactivating the endonuclease is performed for at least 5 minutes, more preferably for at least 10 minutes,

The step of incubating the single contiguous aqueous volume may comprise cycling between a first temperature and a second temperature. The step of incubating the single contiguous aqueous volume may comprise cycling between the first temperature and the second temperature at least 2, at least 3, at least 4, at least 5, at least 6, at least 7, at least 8, at least 9, at least 10, at least 15, at least 20, at least 25, at least 30, at least 35, at least 40, at least 45, at least 50, at least 55, at least 60, at least 65, at least 70, at least 80, at least 90, or at least 100 times, preferably at least 20 times. The step of incubating the single contiguous aqueous volume may comprise cycling between the first temperature and the second temperature less than 40, less than 35, less than 30 times, less than 29, less than 25 times. The step of incubating the single contiguous aqueous volume may comprise cycling between the first temperature and the second temperature 2-100, 5-80, 10-70, 20-60, or 30-60 times. The step of incubating the single contiguous aqueous volume may comprise cycling between the first temperature and the second temperature 2-20, 5-29, 61-100, or 65-80 times.

Rolling circle amplification may be performed without any primers, or in the presence of a primer or multiple primers. For example, the primer may be a synthetic primer. The primers may be random primers. Rolling circle amplification may be performed in the presence of a primase. The primase may be TthPrimPol. Preferably, if the rolling circle amplification is performed without any primers, it is performed in the presence of a primase, such as TthPrimPol. Similarly, if a primer is used during amplification reaction, a primase is not used. The double-stranded DNA molecule may be generated by the rolling circle amplification in vitro under isothermal conditions using a suitable nucleic acid polymerase, such as Phi29 DNA polymerase.

In the methods described herein, the DNA template molecule and/or double-stranded DNA molecule may comprise at least one cleavable target sequence. Preferably, the DNA template molecule and/or double-stranded DNA molecule comprises at least two cleavable target sequences. At least one cleavable target sequence may be 3' (i.e. downstream) of a sequence of interest and at least one cleavable target sequence may be 5' (i.e. upstream) of a sequence of interest.

The cleavable target sequence may be an endonuclease target sequence. Thus, the DNA template molecule and/or double-stranded DNA molecule may comprise at least one endonuclease target sequence. Preferably, the DNA template molecule and/or double-stranded DNA molecule comprise at least two endonuclease target sequences. At least one endonuclease target sequence may be 3' (i.e. downstream) of a sequence of interest and at least one endonuclease target sequence may be 5' (i.e. upstream) of a sequence of interest.

The endonuclease target sequences may be the same or different. Preferably, the at least one endonuclease target sequence is a restriction endonuclease target sequence. Different restriction endonuclease target sequences are known to the skilled person. The cleavable target sequence may be any cleavable target sequence that generates a 5' overhang (preferably a 5' overhang that is at least two nucleotides in length). The cleavable target sequence may be a Type II restriction endonuclease target sequence. The Type II restriction endonuclease target sequence may be a Type IIP restriction endonuclease target sequence (e.g. a EcoRI, Hindlll, BamHI or NotI target sequence) or a Type IIS restriction endonuclease target sequence (e.g. a Bsal, Bbvl, Fokl or Sapl target sequence).

The at least one cleavable sequence (e.g. endonuclease target sequence) may be a native cleavable sequence (i.e. a cleavable sequence present in the template molecule). Alternatively, the at least one cleavable sequence (e.g. endonuclease target sequence) may be introduced to the DNA template molecule and/or double-stranded DNA molecule prior to the production of the protected DNA product.

The endonuclease may be a restriction enzyme endonuclease. The endonuclease may be any endonuclease that generates a 5' overhang (preferably a 5' overhang that is at least two nucleotides in length). The endonuclease may be a restriction endonuclease (such as a Type II restriction endonuclease), RNA-guided DNA endonuclease, nicking endonuclease, meganuclease or homing endonuclease. The Type II restriction endonuclease may be a Type IIP restriction endonuclease (e.g. EcoRI, Hindlll, BamHI or Notl) or a Type IIS restriction endonuclease (e.g. Bsal, Bbvl, Fokl or Sapl).

The DNA template molecule used in the methods described herein may be single-stranded or double-stranded. Preferably, the DNA template molecule is double-stranded. The DNA template molecule and/or double-stranded DNA molecule may be a natural circular DNA molecule. For example, the DNA template molecule and/or double-stranded DNA molecule may be (i) a plasmid, (ii) a minicircle, (iii) a cosmid, (iv) a bacterial artificial chromosome (BAC), or (v) a molecular inversion probe (MIP). The DNA template molecule and/or double-stranded DNA molecule may be an enzymatically produced circular DNA molecule. For example, the DNA template molecule and/or double-stranded DNA molecule may be (i) a circular DNA molecule obtained from recombinase reaction, preferably Cre recombinase reaction, or (ii) a circular DNA molecule obtained from ligase reaction, preferably using the golden gate assembly. The DNA template molecule and/or double-stranded DNA molecule may be an enzymatically produced covalently-closed linear DNA molecule. For example, the DNA template molecule and/or double-stranded DNA molecule may be (i) a DNA molecule processed with TelN protelomerase; or (ii) a DNA molecule generated by ligation of the DNA ends with an adaptor. The DNA template molecule and/or double-stranded DNA molecule may comprise an element that is double-stranded and an element that is single-stranded. For example, the template DNA molecule and/or double-stranded DNA molecule may comprise a double-stranded DNA and a single-stranded hairpin loop.

The DNA template molecule and/or double-stranded DNA molecule may be linear. If the DNA template molecule is linear, prior to amplification (e.g. rolling circle amplification), a DNA template molecule may be circularized to produce a DNA template molecule suitable for use in the methods described herein.

The template DNA molecule and/or double-stranded DNA molecule may comprise a cassette. The cassette may be a mammalian expression cassette. The cassette may further comprise a promoter. The promoter may be a CMV promoter. The cassette may further comprise an enhancer. The cassette may further comprise a reporter gene, such as an eGFP reporter gene or a luciferase reporter gene. The cassette may further comprise a homopolymeric sequence. The cassette may further comprise a LoxP sequence, preferably two LoxP sequences. If the two LoxP sequences are oriented in the same direction, the DNA sequence between the two LoxP sequences is excised as a circular loop of DNA. If the two LoxP sequences are oriented in the opposite direction, the DNA sequence between the two LoxP sequences is inverted. Thus, preferably, the two LoxP sequences are in the same orientation (i.e. the same direction) in the template DNA molecule and/or double-stranded DNA molecule.

The DNA template molecule and/or double-stranded DNA molecule may comprise a homopolymeric sequence at a 5'-end or a 3'-end or both a 5'-end and a 3'-end. The homopolymeric sequence may be added to the DNA template molecule before circularization. The homopolymeric sequence may be a polyA, a polyC, a polyG, or a polyT sequence. The homopolymeric sequence may be between 3-200 nucleotides in length. The homopolymeric sequence may be used to facilitate purification of the linear DNA product, in which case, the homopolymeric sequence may be between 4-12 nucleotides in length, or between 5-10 nucleotides in length. The homopolymeric sequence may be used to improve mRNA expression, in which case, the homopolymeric sequence may be between 10-200 nucleotides in length, preferably between 80-150 nucleotides in length. The homopolymeric sequence may be at least 10, at least 20, at least 30, at least 40, at least 50, at least 60, at least 70, at least 80, at least 90, at least 100, at least 110, at least 120, at least 130, at least 140, at least 150, at least 160, at least 170, at least 180, at least 190, or at least 200 nucleotides in length. Preferably, the homopolymeric sequence is at least 100 nucleotides in length. More preferably still, the homopolymeric sequence is at least 120 nucleotides in length. For example, the homopolymeric sequence may comprise a polyA sequence of at least 120 nucleotides.

The protected DNA product may comprise a cassette, optionally a single cassette. The double-stranded DNA molecule may comprise a cassette, optionally a single cassette. The digested double-stranded DNA molecule may comprise a cassette, optionally a single cassette. The DNA template may comprise a cassette, optionally a single cassette

The term "single cassette" as used herein is intended to encompass a molecule that does not comprise or consist of a plurality of cassettes. That is to say that the protected DNA product may comprise only a single cassette, which may comprise a single coding sequence of a gene of interest. The single cassette may not comprise or consist of a plurality of tandem repeat sequences, and/or concatemeric DNA. The term "single cassette" as used herein is intended to encompass a single copy of the DNA sequence of interest, for example, a single copy of the coding sequence. Thus, the "single cassette" may not encompass a cassette that comprises or consist of multiple copies of the same DNA sequence linked in series. The single cassette may comprise a collection of genes of interest. For example, the single cassette may comprise the sequence for at least two, three, four, or five genes of interest. The genes of interest may not be the same in a single cassette.

The method for producing a protected DNA product may be a cell-free method.

The protected DNA product may be single-stranded or double-stranded. A single-stranded protected DNA product may comprise (or consist of) the extended first strand or the extended second strand.

The protected DNA product may be partially double-stranded and/or partially single-stranded. The protected DNA product may comprise a portion that is double-stranded and a portion that is single-stranded.

The protected DNA product may comprise a spacer. The spacer may be at least 10, at least 20, at least 30, at least 40, at least 50, at least 60, at least 70, at least 80, at least 90, at least 100, at least 125, at least 150, at least 175, or at least 200 base pairs long.

The protected DNA product may comprise an inverted terminal repeat sequence.

The protected DNA product may be at least 50, at least 100, at least 250, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, or at least 15,000 base pairs long. Preferably, the protected DNA product is at least 50 base pairs long.

The double-stranded DNA molecule may be circular or branched.

The double-stranded DNA molecule may not comprise an adaptor. The double-stranded DNA molecule may not comprise a hairpin, a loop or a stem-loop structure.

The double-stranded DNA molecule may be a product of amplification. Preferably, the amplification is rolling circle amplification.

The double-stranded DNA molecule may comprise a double-stranded region that is at least 50, at least 100, at least 250, at least 500, at least 1000, at least 2000, at least 3000, at least 4000, at least 5000, at least 6000, at least 7000, at least 8000, at least 9000, at least 10,000, at least 11,000, at least 12,000, at least 13,000, at least 14,000, or at least 15,000 base pairs long. Preferably, the double-stranded DNA molecule is at least 50 base pairs long.

The double-stranded DNA molecule may comprise a double-stranded region comprising a sequence that is at least 40%, 45%, 50%, 55%, 60%, 65%, 70%, 75%, 80%, 85%, 90%, 95%, or 100% identical to the sequence of the double-stranded DNA molecule.

The digested double-stranded DNA molecule comprises at least one 5' overhang. The digested double-stranded DNA molecule may comprise a blunt end. The digested double-stranded DNA molecule may comprise: a 5' overhang and a blunt end, two 5' overhangs, or a 5' overhang and a 3' overhang. Preferably, the digested double-stranded DNA molecule has two 5' overhangs. The overhang may be in the sense strand and/or the antisense strand of the digested double-stranded DNA molecule.

The protected DNA product may comprise a plurality of nuclease-resistant nucleotides (e.g. phosphorothioated nucleotides) at internal positions in each strand. For example, the protected DNA product may comprise at least 2, at least 4, at least 6, at least 8, at least 10, at least 12, at least 14, at least 16, at least 18, at least 20, at least 30, at least 40, at least 50, at least 60, at least 70, at least 80, at least 90, at least 100, at least 125, at least 150, at least 175, at least 200, at least 250, at least 300, at least 350, at least 400, at least 450, or at least 500 nuclease-resistant nucleotides (e.g. phosphorothioated nucleotides) at internal positions in each strand. The protected DNA product may comprise at least 2 nuclease-resistant nucleotides (e.g. phosphorothioated nucleotides) at internal positions in each strand. The protected DNA product may comprise at least 2 nuclease-resistant nucleotides (e.g. phosphorothioated nucleotides) in the extended first strand and/or the extended second strand. The at least 2 nuclease-resistant nucleotides may be within a region that is 4 or 5 nucleotides from the 3' end of an extended strand.

The nucleotides resistant to exonuclease digestion (i.e. nuclease-resistant nucleotides or protected nucleotides) suitable for use in the methods described herein may be phosphorothioated nucleotides. For example, phosphorothioated nucleotides may be α-S-dATP (i.e. 2'-deoxyadenosine-5'-(α-thio)-triphosphate), α-S-dCTP (i.e. 2'-deoxycytidine-5'-(α-thio)-triphosphate), α-S-dGTP (i.e. 2'-deoxyguanosine-5'-(α-thio)-triphosphate), α-S-dTTP (i.e. 2'-deoxythymidine-5'-(α-thio)-triphosphate), α-S-dUTP (i.e. 2'-deoxyuridine-5'-(α-thio)-triphosphate), and/or uridine 2', 3'-cyclophosphorothioate.

The phosphorothioated nucleotides may be Sp-isomers, Rp-isomers or a mixture of both Sp- and Rp-isomers.

The nucleotides resistant to exonuclease digestion (i.e. nuclease-resistant nucleotides or protected nucleotides) may be 2'-O-methyl nucleotides or 2'-O-methoxyethyl (MOE) nucleotides. For example, the MOE nucleotides may be 2'-O-methoxy-ethyl guanosine, 2'-O-methoxy-ethyl cytidine, 2'-O-methoxy-ethyl adenosine, and/or 2'-O-methoxy-ethyl thymidine.

The nuclease-resistant nucleotides may comprise one or more locked nucleic acids (LNAs).

As used herein, the term "complementary" refers to the pairing of nucleotide sequences according to Watson/Crick pairing rules. For example, a sequence 5'-ATGC-3' has the complementary sequence of 5'- GCAT-3'. A complement sequence can also be a sequence of RNA complementary to the DNA sequence.

After extending the strand(s) 5' to 3' using a polymerase, the protected DNA product comprises one or more nuclease-resistant nucleotides (e.g. phosphorothioated nucleotides) at the 3'-end (or at the 3'-end region) of one or both strands. Most exonucleases, for example exonuclease III, remove nucleotides from the 3'-end of a polynucleotide chain, and the protected DNA product comprises one or more nuclease-resistant nucleotides at the 3'-end of one or both strands. Preferably, the protected DNA product comprises at least one (preferably at least two) phosphorothioated nucleotide(s) at the 3'-end of one or both strands. The protected DNA product may comprise at least one (preferably at least two) phosphorothioated nucleotide(s) at the 3'-end and at least one (preferably at least two) phosphorothioated nucleotide(s) at the 5'-end of one or both strands.

The protected DNA molecule may comprise one type of phosphorothioated nucleotide e.g. α-S-dATP, α-S-dCTP, α-S-dGTP or α-S-dTTP.

The protected DNA molecule may comprise at least two types of phosphorothioated nucleotides. For example, the at least two types of phosphorothioated nucleotides are: α-S-dATP and α-S-dCTP, α-S-dATP and α-S-dGTP, α-S-dATP and α-S-dTTP, α-S-dCTP and α-S-dGTP, α-S-dCTP and α-S-dTTP, or α-S-dGTP and α-S-dTTP.

The protected DNA molecule may comprise at least three types of phosphorothioated nucleotides. For example, the at least three types of phosphorothioated nucleotides are:
(a) α-S-dATP, α-S-dCTP and α-S-dGTP;
(b) α-S-dATP, α-S-dCTP and α-S-dTTP;
(c) α-S-dATP, α-S-dGTP and α-S-dTTP; or
(d) α-S-dCTP, α-S-dGTP and α-S-dTTP.

The protected DNA molecule may comprise at least four types of phosphorothioated nucleotides. For example, the at least four types of protected nucleotides are α-S-dATP, α-S-dCTP, α-S-dGTP and α-S-dTTP.

The protected DNA product produced by the methods described herein has enhanced resistance to nuclease (e.g. exonuclease) digestion. The protected DNA product may be resistant to digestion by 3'-5' exonuclease activity. The protected DNA product may not be resistant to digestion by 5'-3' exonuclease activity.

The protected DNA product may comprise a plurality of nuclease-resistant nucleotides (e.g. phosphorothioated nucleotides). For example, the protected DNA product may comprise at least 2, at least 3, at least 4, at least 5, at least 6, at least 7, at least 8, at least 9, at least 10, at least 11, at least 12, at least 13, at least 14, at least 15 or at least 16 nuclease-resistant nucleotides (e.g. phosphorothioated nucleotides) in one or both strands.

The extended portion of the first and/or second strands may comprise at least 5%, at least 10%, at least 20%, at least 30%, at least 40%, at least 50%, at least 60%, at least 70%, at least 80%, at least 90%, at least 95%, or at least 100% of nuclease-resistant nucleotides. Thus, the extended portion of the first and/or second strands may consist of nuclease-resistant nucleotides. The extended portion(s) refers to the at least one (preferably at least two) nucleotide(s) added to the digested double-stranded DNA molecule.

The protected DNA product may be linear.

### 2. Protected DNA products

The invention provides a protected DNA product as described herein.

The invention provides a protected DNA product produced or obtainable by the methods for producing a protected DNA product as described herein.

### 3. Methods for transcription and protein expression

The invention provides a method for in vitro transcription of a protected DNA product, wherein the method comprises contacting the protected DNA product produced or obtainable by the methods described herein with a (RNA) polymerase and producing a transcription product encoded by the protected DNA product.

The invention provides a method for in vitro transcription of a protected DNA product, wherein the method comprises:
(a) producing a protected DNA product by any of the methods described herein;
(b) contacting the protected DNA product with a (RNA) polymerase; and
(c) producing a transcription product encoded by the protected DNA product.

The invention provides a method for producing a protein, wherein the method comprises introducing the protected DNA product, produced or obtainable by the methods described herein, into a cell (e.g. a prokaryotic cell or a eukaryotic cell) or a cell-free expression system to generate a protein encoded by the protected DNA product.

The invention provides a method for producing a protein, wherein the method comprises:
(a) producing a protected DNA product by any of the methods described herein; and
(b) introducing the protected DNA product into a cell (e.g. a prokaryotic cell or a eukaryotic cell) or a cell-free expression system to generate a protein encoded by the protected DNA product.

The cell-free expression system may originate from (or be derived from) a prokaryotic cell or eukaryotic cell. For example, the cell-free expression system may originate from (or be derived from) rabbit reticulocytes, wheat germ or *Escherichia coli.*

The cell may be a prokaryotic cell or a eukaryotic cell. The cell may be an animal cell, such as mammal cell (e.g. a human cell), a fungal cell, a cell of a micro-organism (e.g. a prokaryotic cell or a eukaryotic cell), or a plant cell. Preferably, the cell is a human cell.

The protected DNA product may comprise a cassette. The desired protein might be encoded by the cassette. The step of introducing the protected DNA product into a cell may be performed in vivo or in vitro. The nuclease-resistant nucleotides (i.e. protected nucleotides) may be any protected nucleotides described herein.

### 4. Methods for cell transfection and cell transfection compositions

The invention provides a method for cell transfection of a protected DNA product produced or obtainable by any of the methods described herein, into a cell.

The invention provides a method for cell transfection of a protected DNA product into a cell, wherein the method comprises:
(a) producing a protected DNA product by any of the methods described herein;
(b) contacting a cell with the protected DNA product; and
(c) transfecting the protected DNA product into the cytosol of the cell.

The invention provides a cell transfection composition comprising a protected DNA product produced by (or obtainable by) the methods described herein.

The cell transfection composition may or may not comprise a carrier e.g. an agent or a formulation. Preferably, the carrier promotes accumulation of the protected DNA product at a target site and/or protects the protected DNA product from undesirable interactions with biological milieu components and/or protects the protected DNA product from metabolism and/or degradation.

The invention further provides a cell transfection method comprising contacting (in vitro) a cell to be transfected with a protected DNA product produced or obtainable by the methods of the invention, and wherein the protected DNA product is transfected into the cytosol of the cell.

The cell or cells to be transfected may be provided in a cell culture medium (e.g. in a Petri dish, culture vessel or well, etc). The protected DNA product may be added directly to the cell culture medium or the cells may be added to a solution, such as saline, a buffered solution or a cell culture medium, comprising the protected DNA product.

The carrier may be a viral carrier or a non-viral carrier. Viral carriers include a lentiviral vector, an adenoviral vector, a retroviral vector, or an adeno associate viral vector for delivery of the protected DNA product. Non-viral carriers (or vectors) include complexing the protected DNA product with a cationic agent such as a cationic cell penetrating peptide (CPP); a DNA-binding cationic component, such as a polylysine chain; a cationic polymer or dendrimer e.g. polyethylenimine (PEI), poly-D,L-lactide-co-glycolide (PLGA), and a block copolymer of PEG and polylysine, and/or a cationic lipid (e.g. lipofectamine).

The carrier may be a small molecule (e.g., cholesterol, bile acid, and/or lipid), polymer, protein (e.g. an antibody), and/or aptamer (e.g. RNA) that is conjugated to the protected DNA product. The carrier may be a nanoparticulate formulation used to encapsulate the protected DNA product.

The carrier may be a modification of the protected DNA product with a targeting ligand (e.g. an antibody), a peptide, a small molecule (e.g. folic acid and/or biotin), or a polymer present in extracellular matrix (e.g. hyaluronic acid and/or chondroitin sulphate), or a hydrophobic modification of the double-stranded nucleic acid molecule (e.g. using cholesterol and/or α-tocopherol).

The cell transfection composition may or may not comprise an agent selected from a photosensitizing agent and/or a radical initiator e.g. a photoinitiator. Preferably, this agent improves the function of the protected DNA product at a target site and/or protects the protected DNA product from metabolism and/or degradation.

The invention further provides a cell obtainable by the methods of the invention. Thus, the cell may comprise a protected DNA product produced or obtainable by the methods of the invention.

The invention further provides a cell transfected with a protected DNA product produced or obtainable by the methods of the invention.

The cell may be an animal cell, such as mammal cell (e.g. a human cell), a fungal cell, a cell of a micro-organism (e.g. a prokaryotic cell or a eukaryotic cell), or a plant cell. Preferably, the cell is a human cell.

The step of contacting the cell with a protected DNA product may be performed in vivo. For example, the protected DNA product may be administered to an organism (e.g. a subject) in need thereof. The organism (e.g. the subject) may be an animal, such as mammal (e.g. a human), a fungus, a micro-organism, or a plant.

Any or all of the protected DNA products described herein may be delivered to a cell via delivery particles such as liposomes, nanoparticles, exosomes, macrovesicles, viral or non-viral vectors. Any or all of the protected DNA products described herein may be delivered to a cell using a gene-gun. Any or all of the protected DNA products described herein may be delivered to a cell by electroporation. Any or all of the protected DNA products described herein may be delivered to a cell by hydrodynamic needle. The protected DNA products described herein may be delivered to a cell without a carrier.

The nanoparticle is preferably a self-assembled nanoparticle. The nanoparticle may be a nanoparticle which is produced by a process in which pre-existing components (e.g. a lipid component, a DNA product described herein) form an organized structure as a consequence of specific, local interactions among the components themselves, without external direction.

The protected DNA product and the lipid component may reversibly interact to form a self-assembled nanoparticle. The protected DNA product and the lipid component may reversibly interact in the self-assembled nanoparticle through intermolecular forces. The protected DNA product and the lipid component may reversibly interact in the self-assembled nanoparticle through non-covalent interactions. The protected DNA product and the lipid component may reversibly interact in the self-assembled nanoparticle through hydrogen bonds, van der Waals, hydrophobic, and/or electrostatic interactions. The protected DNA product and the lipid component may not be conjugated or linked by forces other than inter-molecular forces in the self-assembled nanoparticle.

### 5. Pharmaceutical compositions and methods for producing pharmaceutical compositions

The invention provides a pharmaceutical composition comprising a protected DNA product described herein, and a pharmaceutically acceptable carrier or excipient.

The invention provides a pharmaceutical composition comprising a protected DNA product produced or obtainable by the methods described herein, and a pharmaceutically acceptable carrier or excipient.

The invention provides a method for producing a pharmaceutical composition comprising the protected DNA product, wherein the method comprises performing the methods described herein to produce the protected DNA product and formulating the resulting protected DNA product with a pharmaceutically acceptable carrier or excipient.

Thus, the invention provides a method for producing a pharmaceutical composition, wherein the method comprises:
(a) producing a protected DNA product by any of the methods described herein;
(b) formulating the protected DNA product with a pharmaceutically acceptable carrier or excipient.

The pharmaceutical composition may be formulated as pills, tablets or capsules combined with one or more pharmaceutically acceptable solid carriers or as a solution in one or more pharmaceutically acceptable solvents, or as an emulsion, suspension or dispersion in one or more pharmaceutically acceptable solvents or carriers. The formulation may also include other pharmaceutically acceptable excipients such as stabilizers, anti-oxidants, binders, colouring agents or emulsifying or taste-modifying agents and extended release formulations.

The pharmaceutical composition may be administered orally, topically, parenterally or transdermally or by inhalation. The pharmaceutical composition may be administered by injection or intravenous infusion using suitable sterile solutions. Topical dosage forms may be creams, ointments, patches, or similar vehicles suitable for transdermal and topical dosage forms.

The pharmaceutical composition may be dissolved or suspended in a liquid vehicle or formulated as a granule (a small particle or grain), a pellet (a small sterile solid mass consisting of a highly purified composition, with or without excipients, made by the formation of granules, or by compression and moulding), or a pellet coated extended release (a solid dosage form in which the composition itself is in the form of granules to which varying amounts of coating have been applied, and which releases the composition in such a manner to allow a reduction in dosing frequency as compared to that composition presented as a conventional dosage form).

Other forms of pharmaceutical compositions include pills (a small, round solid dosage form containing the composition intended for oral administration), powder (an intimate mixture of dry, finely divided composition with one or more pharmaceutically acceptable additives that may be intended for internal or external use), elixir (a clear, pleasantly flavoured, sweetened hydroalcoholic liquid containing dissolved composition; it is intended for oral use), chewing gum (a sweetened and flavoured insoluble plastic material of various shapes which when chewed, releases the composition into the oral cavity), syrup (an oral solution containing the composition and high concentrations of sucrose or other sugars; the term has also been used to include any other liquid dosage form prepared in a sweet and viscid vehicle, including oral suspensions), tablet (a solid dosage form containing the composition with or without suitable diluents), tablet chewable (a solid dosage form containing the composition with or without suitable diluents that is intended to be chewed, producing a pleasant tasting residue in the oral cavity that is easily swallowed and does not leave a bitter or unpleasant after-taste), tablet coated or tablet delayed release, tablet dispersible, tablet effervescent, tablet extended release, tablet film coated, or tablet film coated extended release where the tablet is formulated in such manner as to make the contained composition available over an extended period of time following ingestion.

In other forms of pharmaceutical compositions, a tablet for solution, tablet for suspension, tablet multilayer, tablet multilayer extended release may be provided, where the tablet is formulated in such manner as to allow at least a reduction in dosing frequency as compared to that composition presented as a conventional dosage form. A tablet orally disintegrating, tablet orally disintegrating delayed release, tablet soluble, tablet sugar coated, osmotic, and the like are also suitable.

The oral dosage form pharmaceutical composition may contain, in addition to the composition, one or more inactive pharmaceutical ingredients such as diluents, solubilizers, alcohols, binders, controlled release polymers, enteric polymers, disintegrants, excipients, colorants, flavorants, sweeteners, antioxidants, preservatives, pigments, additives, fillers, suspension agents, surfactants (e.g., anionic, cationic, amphoteric and nonionic), and the like. Various FDA-approved topical inactive ingredients are found at the FDA's "The Inactive Ingredients Database" that contains inactive ingredients specifically intended as such by the manufacturer.

As used herein, injectable and infusion dosage forms include, but are not limited to, a liposomal injectable, which either consists of or forms liposomes (a lipid bilayer vesicle usually composed of phospholipids which is used to encapsulate the composition); an injection, which includes a sterile preparation intended for parenteral use; an emulsion injection, which includes an emulsion consisting of a sterile, pyrogen-free preparation intended to be administered parenterally; or a lipid complex injection.

For example, the protected DNA product can be administered by intratympanic injection (e.g. into the middle ear) and/or injections into the outer, middle, and/or inner ear. Such methods are routinely used in the art, for example, for the administration of steroids and antibiotics into human ears. Injection can be, for example, through the round window of the ear or through the cochlear capsule.

Other forms of pharmaceutical composition include a powder for solution injection, which is a sterile preparation intended for reconstitution to form a solution for parenteral use; a powder for suspension injection that is a sterile preparation intended for reconstitution to form a suspension for parenteral use; a powder lyophilized for liposomal suspension injection, which is a sterile freeze dried preparation intended for reconstitution for parenteral use which has been formulated in a manner that would allow liposomes (a lipid bilayer vesicle usually composed of phospholipids which is used to encapsulate the composition, either within a lipid bilayer or in an aqueous space) to be formed upon reconstitution; or a powder lyophilized for solution injection, wherein lyophilization ("freeze drying") is a process which involves the removal of water from products in the frozen state at extremely low pressures.

A suspension injection comprises a liquid preparation, suitable for injection, which consists of solid particles dispersed throughout a liquid phase in which the particles are not soluble that can also consist of an oil phase dispersed throughout an aqueous phase, or vice-versa. A suspension liposomal injection comprises a liquid preparation, suitable for injection, which consists of an oil phase dispersed throughout an aqueous phase in such a manner that liposomes (a lipid bilayer vesicle usually composed of phospholipids which is used to encapsulate the composition, either within a lipid bilayer or in an aqueous space) are formed. A suspension sonicated injection comprises a liquid preparation, suitable for injection, which consists of solid particles dispersed throughout a liquid phase in which the particles are not soluble. In addition, the product is sonicated while a gas is bubbled through the suspension, and this results in the formation of microspheres by the solid particles.

In another mode of administration, the pharmaceutical composition can be administered in situ, via a catheter or pump. A catheter or pump can, for example, direct the composition into the target location.

The parenteral carrier system may include one or more pharmaceutically suitable excipients, such as solvents and co-solvents, solubilizing agents, wetting agents, suspending agents, thickening agents, emulsifying agents, chelating agents, buffers, pH adjusters, antioxidants, reducing agents, antimicrobial preservatives, bulking agents, protectants, tonicity adjusters, and special additives. Formulations suitable for parenteral administration conveniently comprise a sterile oily or aqueous preparation of the composition which is preferably isotonic with the blood of the recipient but this is not essential.

As used herein, inhalation dosage forms include, but are not limited to, an aerosol (a product that is packaged under pressure and contains the composition which is released upon activation of an appropriate valve system intended for topical application to the skin as well as local application into the nose (nasal aerosols), mouth (lingual and sublingual aerosols), or lungs (inhalation aerosols)). A foam aerosol is a dosage form containing the composition, surfactants, aqueous or nonaqueous liquids, and propellants, whereby if the propellant is in the internal (discontinuous) phase (i.e., of the oil-in-water type), a stable foam is discharged, and if the propellant is in the external (continuous) phase (i.e., of the water-in-oil type), a spray or a quick-breaking foam is discharged. A metered aerosol is a pressurized dosage form consisting of metered dose valves which allow for the delivery of a uniform quantity of spray upon each activation. A powder aerosol is a product that is packaged under pressure and contains the composition, in the form of a powder that is released upon activation of an appropriate valve system. An aerosol spray is an aerosol product which utilizes a compressed gas as the propellant to provide the force necessary to expel the product as a wet spray and being applicable to solutions of the composition in aqueous solvent(s).

A transdermal dosage form may include, but is not limited to, a patch (a drug delivery system that often contains an adhesive backing that is usually applied to an external site on the body, whereby the ingredients (including the composition) either passively diffuse from, or are actively transported from, some portion of the patch, and whereby depending upon the patch, the ingredients (including the composition are either delivered to the outer surface of the body or into the body. Various types of transdermal patches such as matrix, reservoir and others are known in the art.

A topical dosage form may include various dosage forms known in the art such as lotions (an emulsion, liquid dosage form, whereby this dosage form is generally for external application to the skin), lotion augmented (a lotion dosage form that enhances composition delivery, whereby augmentation does not refer to the strength of the composition in the dosage form), gels (a semisolid dosage form that contains a gelling composition to provide stiffness to a solution or a colloidal dispersion, whereby the gel may contain suspended particles) and ointments (a semisolid dosage form, usually containing less than 20% water and volatiles and greater than 50% hydrocarbons, waxes, or polyols as the vehicle, whereby this dosage form is generally for external application to the skin or mucous membranes). Further embodiments include ointment augmented (an ointment dosage form that enhances composition delivery, whereby augmentation does not refer to the strength of the composition in the dosage form), creams (an emulsion, semisolid dosage form, usually containing greater than 20% water and volatiles and/or less than 50% hydrocarbons, waxes, or polyols may also be used as the vehicle, whereby this dosage form is generally for external application to the skin or mucous membranes) and cream augmented (a cream dosage form that enhances composition delivery, whereby augmentation does not refer to the strength of the composition in the dosage form). As used herein, an "emulsion" refers to a dosage form consisting of a two-phase system comprised of at least two immiscible liquids, one of which is dispersed as droplets, internal or dispersed phase, within the other liquid, external or continuous phase, generally stabilized with one or more emulsifying agents, whereby emulsion is used as a dosage form term unless a more specific term is applicable (e.g. cream, lotion, ointment). Further embodiments include suspensions (a liquid dosage form that contains solid particles dispersed in a liquid vehicle), suspension extended release, pastes (a semisolid dosage form, containing a large proportion, 20-50%, of solids finely dispersed in a fatty vehicle, whereby this dosage form is generally for external application to the skin or mucous membranes), solutions (a clear, homogeneous liquid dosage form that contains one or more chemical substances dissolved in a solvent or mixture of mutually miscible solvents), and powders.

The topical dosage form composition contains the composition and one or more inactive pharmaceutical ingredients such as excipients, colorants, pigments, additives, fillers, emollients, surfactants (e.g., anionic, cationic, amphoteric and nonionic), penetration enhancers (e.g., alcohols, fatty alcohols, fatty acids, fatty acid esters and polyols), and the like. Various FDA-approved topical inactive ingredients are found at the FDA's "The Inactive Ingredients Database" that contains inactive ingredients specifically intended as such by the manufacturer.

### 6. Uses and Applications

The invention provides a use of a protected DNA product as described herein in the production of viral or non-viral delivery system.

The invention provides a use of a protected DNA product in the production of viral or non-viral delivery system, wherein the protected DNA product is produced or obtainable by performing the method described herein.

The invention provides a viral or non-viral delivery system comprising a protected DNA product as described herein. The invention provides a viral or non-viral delivery system comprising a protected DNA product, wherein the protected DNA product is produced or obtainable by performing the method described herein.

### Viral vectors

Methods of producing viral vectors, such as AAV vectors, are known in the art. The most widely used method involves the co-transfection of HEK293 by three bacterial plasmids. The first plasmid encodes the *Rep* and Cap element, the second plasmid is a helper plasmid, while the third plasmid encodes the genetic payload of interest with inverted terminal repeats (ITRs). There are several issues surrounding the use of plasmids for viral preparation (e.g. AAV), namely: 1) the production of the plasmids is time consuming and costly, 2) there is a difficulty in the propagation of ITR sequences in *E.coli;* 3) the incorporation of the plasmid backbone into the viral capsid (e.g. AAV capsid) might be challenging (e.g. issues with antibiotic resistance markers). Methods of producing viral vectors may be using other cell lines. For example, a cell line suitable for production of viral vectors may be a Vero cell, or any other stable cell line. Together these represent the main bottleneck in viral vector production (e.g. AAV production). Thus, the methods described herein provide a protected DNA product suitable for use in production of viral vectors. The protected DNA product overcomes the above issues with plasmid vectors.

The invention provides a method of producing a viral vector, the method comprising introducing the protected DNA product produced or obtainable by the methods described herein into a cell under conditions such that the viral vector is produced. The protected DNA product may encode at least one element required for the production of the viral vector. For example, the protected DNA product may encode Rep and/or Cap elements. The protected DNA product may encode the helper plasmid elements. The protected DNA product may encode Rep, Cap and helper plasmid elements. The protected DNA product may encode a transgene. The method may be an in vivo or in vitro method. The cell may be an animal cell, preferably mammal cell, such as human cell (e.g. HEK293T, HEK293, CAP, CAP-T, or CHO). The cell may be a cell cultured in vitro in a tissue culture cell line.

Preferably, the vector is an AAV vector or lentivirus vector.

The invention also provides a method of delivering the viral vector (e.g. the protected DNA product) to a cell, comprising contacting the viral vector produced by the methods described herein with the cell. The cell may be an animal cell, preferably mammal cell, such as human cell.

The invention also provides a cell obtainable by the methods described herein.

### Non-viral vectors

Methods of producing non-viral vectors, are known in the art. Use of non-viral vectors over viral vectors has several advantages, including the opportunity of repeat dosing due to their non-immunogenicity, an unlimited packaging capacity, and low associated toxicity. Most methods for producing non-viral vectors use plasmid DNA. Thus, the protected DNA product produced or obtainable by the methods described herein is suitable for use in production of non-viral vectors. The protected DNA product produced or obtainable by the methods of the invention overcomes the issues of using plasmid vectors in non-viral vector preparation. For example, the protected DNA product, unlike plasmid DNA, does not comprise a bacterial backbone, allowing more transgene copies per mg of DNA. In addition, the protected DNA product does not comprise antibiotic resistant genes and bacterial contaminants. Moreover, the protected DNA product provides a prolonged transgene expression (due to the presence of exonuclease-resistant nucleotides), and a more cost-effective process of non-viral vector production.

The invention provides a method of delivering the non-viral vector to a cell, comprising contacting the non-viral vector comprising the protected DNA product with the cell. The cell may be an animal cell, preferably mammal cell, such as human cell.

The invention also provides a cell obtainable by the methods described herein.

### General therapeutic and diagnostic uses

The protected DNA product produced by the methods described herein is particularly suitable for use in therapy. The invention provides a protected DNA product as described herein for use in therapy. The invention provides a protected DNA product obtainable by the method described herein for use in therapy. The protected DNA product may encode a sequence of a therapeutic protein, a part of a vaccine, or an element of a genetic engineering mechanism, which is used to treat a disease or infection in a subject.

The invention further provides the protected DNA product as described herein for use as a medicament. The invention further provides the protected DNA produced by the methods described herein for use as a medicament. The invention further provides the protected DNA product obtainable by the methods described herein for use as a medicament. The invention also provides the use of a protected DNA product as described herein for the manufacture of a medicament for treating a disease. The invention also provides the use of a protected DNA product produced by the methods described herein for the manufacture of a medicament for treating a disease. The invention also provides the use of a protected DNA product obtainable by the methods described herein for the manufacture of a medicament for treating a disease.

The protected DNA product may encode a sequence of a therapeutic protein, a part of a vaccine, or an element of a genetic engineering mechanism, which is used to treat a disease or infection in a subject.

The invention further provides the protected DNA product produced by the methods described herein for use in treating a disease.

The invention also provides a method of treating a disease in a subject comprising administering to the subject a protected DNA product described herein. The invention also provides a method of treating a disease in a subject comprising administering to the subject the protected DNA product produced by the methods described herein. The invention also provides a method of treating a disease in a subject comprising administering to the subject the protected DNA product obtainable by the methods described herein. Preferably, the amount of the protected DNA product administered to the subject is a therapeutic active amount.

The protected DNA product described herein may be used to treat any disease or disorder. For example, the protected DNA product may be used to treat one or more of the diseases and/or disorders selected from genetic disorders (e.g. monogenic disorders), cancer, HIV, other viral infections (e.g. infection caused by coronavirus (e.g. COVID-19), hepatitis A, hepatitis B, herpes simplex virus type 2, influenza, measles and/or respiratory syncytial virus), neurodegenerative diseases (e.g. Parkinson's disease and/or polyglutamine diseases such as Huntington's disease), ocular diseases (e.g. macular degeneration) and liver failure. Preferably, the protected DNA product is used to treat a genetic disorder. More preferably still, the protected DNA product is used to treat a monogenic disorder. For example, the protected DNA product, may be used to treat sickle cell anaemia, cystic fibrosis, Huntington disease, and Duchenne's Muscular Dystrophy, Haemophilia A, α1-antitrypsin deficiency, primary ciliary dyskinesia, or respiratory distress syndrome of prematurity.

A subject treated with the protected DNA product may receive the protected DNA product in the form of any of the pharmaceutical compositions described herein.

A subject treated with the protected DNA product may receive the protected DNA product in combination with other forms of treatment for the disorder concerned, including treatment with drugs generally used for the treatment of the disorder. The drugs may be administered in one or several dosage units. The skilled person (e.g. a medical practitioner) is well able to determine an appropriate dosage regimen for the subject according to the subject's specific circumstances.

As used herein, "administering" means introducing the protected DNA product into the subject's body as described in more detail above (see "Methods for producing a pharmaceutical composition"). Examples include but are not limited to oral, topical, buccal, sublingual, pulmonary, transdermal, transmucosal, as well as subcutaneous, intraperitoneal, intravenous, and intramuscular injection or in the form of liquid or solid doses via the alimentary canal.

As used herein, the phrase "a therapeutically active amount" means an amount of the protected DNA product that, when administered to a subject for treating a disease, is sufficient to affect such treatment of the disease. A "therapeutically active amount" will vary depending, for instance, on factors such as the specific product used, the severity of subject's disease, the age and relative health of the subject and the route and form of administration. Determining the relevant therapeutically active amount for a specific subject based on such factors is routine for the person skilled in the art (e.g. an attending medical practitioner). Treatment of a disease as described herein should be understood to mean an improvement in one of more of the symptoms of a disease.

The invention also provides the use of the protected DNA product as described herein in a method of diagnosing a disease and/or a disorder. The invention also provides the use of the protected DNA product produced or obtainable by the methods described herein in a method of diagnosing a disease and/or a disorder.

The invention provides the use of the protected DNA product in the "in vitro" diagnosis of a disease. The invention provides the use of the protected DNA product obtainable by the methods described herein in the "in vitro" diagnosis of a disease.

The invention also provides the protected DNA product for use in a method of diagnosis "in vivo" of a disease.

The method may be used to diagnose any disease and/or disorder. For example, the diseases and/or disorders may be selected from genetic disorders (e.g. monogenic disorders), cancer, HIV, other viral infections (e.g. infection caused by coronavirus (e.g. COVID-19), hepatitis A, hepatitis B, herpes simplex virus type 2, influenza, measles and/or respiratory syncytial virus), neurodegenerative diseases (e.g. Parkinson's disease and/or polyglutamine diseases such as Huntington's disease), ocular diseases (e.g. macular degeneration) and liver failure. Preferably, the protected DNA product is used to diagnose a genetic disorder. More preferably still, the protected DNA product is used to diagnose a monogenic disorder. For example, the protected DNA product may be used to diagnose sickle cell anaemia, cystic fibrosis, Huntington disease, and Duchenne's Muscular Dystrophy, Haemophilia A, α1-antitrypsin deficiency, primary ciliary dyskinesia, or respiratory distress syndrome of prematurity.

The diagnostic and treatment methods described herein may be in vitro methods or in vivo methods.

The method of diagnosis may rely on the detection and/or quantification of the protected DNA product.

To facilitate detection and/or quantification of the protected DNA product, the protected DNA product may be attached or bound to a functional portion. The functional portion may be any functional portion described herein. For example, the functional portion may be a probe. The functional portion may comprise a fluorophore, a radioactive compound or a barcode. The functional portion may be a protein, for example, an antibody.

The diagnosis may rely on the detection of a signal corresponding to the presence, absence and/or level of the protected DNA product. For example, the signal may be measured by flow cytometry and/or fluorescence-activated cell sorting of a protected DNA product attached to a fluorescent probe.

The protected DNA product may be detected by being bound to a capture moiety, for example in a lateral flow assay. In this example, the functional portion is a protein, for example, an antibody specific for the capture moiety. The capture of the antibody attached to the protected DNA product may produce a visual signal (e.g. a band of a different colour).

The invention also provides a method that combines diagnosis of a disease with a treatment of the disease.

### Cell therapy

The products produced by the methods of the invention may be substantially less contaminated than plasmid DNA. In addition, the products produced by the methods of the invention are very simple in nature (e.g. no bacterial backbone), which means that they are typically easy to work with. The pure and simple nature of the products described herein makes them particularly suitable for use in cell therapy. For example, a cell comprising the protected DNA product may be injected or otherwise transplanted into a patient to cause a desired effect. The cell (or cells) may be capable of fighting cancer cells, for example, via cell-mediated immunity in the course of immunotherapy. The cell (or cells) may be grafted to regenerate diseased tissues.

The invention provides the protected DNA product described herein for use in cell therapy. The invention provides the protected DNA product produced or obtainable by the methods described herein for use in cell therapy.

The invention provides the protected DNA product described herein for use in cell therapy. The invention provides the protected DNA product produced or obtainable by the methods described herein for use in cell therapy.

Preferably, cell therapy is ex-vivo cell therapy. The cell may be an animal cell, preferably mammal cell, such as human cell.

The invention also provides a cell obtainable by any methods described herein. The cell may be suitable for use in cell therapy.

### Vaccines

The protected DNA products produced by the methods described herein are particularly suitable for use in vaccine production. A vaccine may comprise a protected DNA product described herein. A vaccine may comprise a protected DNA product produced or obtainable by the methods described herein. Alternatively, the protected DNA product may be used to produce a vaccine, preferably an mRNA-based vaccine. For example, vaccines such as the BioNTech and Moderna mRNA vaccines against severe acute respiratory syndrome coronavirus 2 (SARS-CoV-2).

Thus, the invention provides the use of the protected DNA product described herein in the production of a vaccine. The invention also provides the use of the protected DNA product produced or obtainable by the methods described herein in the production of a vaccine.

The protected DNA product may encode an antigen, which may cause an immune response in a subject. The subject may be human. Preferably, the antigen is encoded on the cassette.

### CAR-T cells

The invention provides the use of a protected DNA product described herein in the production of a CAR-T cell. The invention provides the use of the protected DNA product produced or obtainable by the methods described herein in the production of a CAR-T cell.

The invention provides a method for producing a genetically engineered CAR-T cell comprising: (a) introducing the protected DNA product described herein into a T cell; and (b) expressing a gene of interest encoding by the protected DNA product. Preferably, the gene of interest is a tumour-specific CAR.

The invention provides a method for producing a genetically engineered CAR-T cell comprising: (a) introducing the protected DNA product obtainable by the methods described herein into a T cell; and (b) expressing a gene of interest encoding by the protected DNA product. Preferably, the gene of interest is a tumour-specific CAR.

The method may further comprise, before step (a) (i.e. introducing the protected DNA product into a T cell), a step of removing mononuclear cells from a patient. Preferably, the step of removing is performed using leukapheresis. Preferably the mononuclear cells are T cells. The method may further comprise, after step (b) (i.e. expressing a gene of interest), a step of returning the genetically engineered cells to the patient.

The invention also provides an engineered T cell obtainable by any methods described herein. The engineered T cell may be suitable for use in CAR T-cell therapy.

### CRISPR delivery

The products produced by the methods described herein are particularly suitable for use in delivery of CRISPR machinery to cells, for example in cell therapy or in vivo therapy.

Various different cargos and delivery vehicles are used commonly for delivery of CRISPR machinery, including physical delivery methods (e.g. microinjection; electroporation), viral delivery methods (e.g. adeno-associated virus (AAV); full-sized adenovirus and lentivirus), and non-viral delivery methods (e.g. liposomes; polyplexes; gold particles).

The protected DNA product may comprise a gene sequence encoding any component of the CRIPSR machinery. The protected DNA product may encode all components of the CRIPSR machinery.

The protected DNA product may comprise (or encode) a repair template (or editing template). The repair template (or editing template) may be for editing genomes, for example, using the CRISPR-Cas system. The repair template (or editing template) may comprise or consist of a homology region (e.g. homology arm) which is homologous to the desired DNA region (i.e. target molecule). The repair template (or editing template) may be for use in CRISPR-Cas mediated homology directed repair (HDR). The repair template (or editing template) may be used to repair a target molecule having a strand break (e.g. a single stranded break or a double stranded break). The strand break may be created by a nuclease of the CRISPR system (e.g. Cpf1 or Mad7). The repair template (or editing template) may introduce at least one mutation (e.g. an insertion, deletion, and/or substitution) in the desired DNA region (i.e. target molecule). The repair template (or editing template) may be at least 10, 20, 30, 40, 50, 100, 200, 300, 400, 500, 600, 700, 800, 900, 1000, 1500, 2000, 2500, 3000, 3500, 4000, 4500, 5000, 5500, 6000, 6500, 7000, 7500, 8000, 8500, 9000, 9500, or 10000 base pairs long. The protected DNA product encoding the repair template may be delivered to a cell by a nanoparticle, a non-viral vector or a viral vector, or without the aid of any carrier. The protected DNA product encoding the repair template may be delivered to a cell by electroporation. The protected DNA product encoding the repair template may be delivered to a cell by hydrodynamic needle.

The protected DNA product may comprise (or further comprise) a gene sequence encoding a nuclease protein of a CRISPR system (e.g. Cpf1 or Mad7), and/or a guide RNA. The protected DNA product may comprise (or further comprise) a gene sequence encoding a nuclease protein of a CRISPR system (e.g. Cpf1, or Mad7). The protected DNA product may comprise (or further comprise) a gene sequence encoding a guide RNA. The protected DNA product may comprise (or further comprise) a gene sequence encoding a nuclease protein of a CRISPR system (e.g. Cpf1, or Mad7) and a guide RNA. The protected DNA product may comprise (or further comprise) a gene sequence encoding a genomic target to be modified (e.g. a spacer). The protected DNA product may be ligated to a vector. The vector may comprise a sequence of some of the components of the CRIPSR system. The vector may comprise a sequence of guide RNA or a sequence of a part of the guide RNA. If the vector comprises a sequence of a part of the guide RNA, the protected DNA product may comprise the missing sequence part of the guide RNA, so that upon ligation, the ligated vector comprises a full sequence of a guide RNA. The nuclease of the CRISPR system and guide RNA may be encoded on a single vector or on two different vectors. The protected DNA product may encode the nuclease of the CRISPR system and guide RNA. One protected DNA product may encode the nuclease of the CRISPR system, and the other protected DNA product may encode guide RNA.

The protected DNA product may be for use in CRISPR-Cas mediated repair by recombination, homology-directed repair, or non-homologous end joining.

If the nuclease of the CRISPR system and the guide RNA are encoded by a different protected DNA product, they may be part of a different or the same delivery mechanism. For example, the protected DNA product encoding the nuclease of the CRISPR system may be delivered to a cell by a first nanoparticle, non-viral vector or viral vector, whereas the protected DNA product encoding the guide RNA may be delivered to a cell by a second nanoparticle, a non-viral vector, or viral vector. For example, the protected DNA product encoding the nuclease of the CRISPR system and the protected DNA product encoding the guide RNA may be delivered to a cell by the same nanoparticle, non-viral vector or viral vector.

If the nuclease of the CRISPR system and the guide RNA are encoded by the same protected DNA product (or by a vector that comprises the protected DNA product) they may be part of the same delivery mechanism. For example, the protected DNA product, or the vector, encoding the nuclease of the CRISPR system and the guide RNA may be delivered to a cell by a nanoparticle, a non-viral vector or a viral vector.

The protected DNA product encoding the nuclease of the CRISPR system and the protected DNA product encoding the guide RNA may be delivered to a cell by electroporation. The protected DNA product encoding the nuclease of the CRISPR system and the protected DNA product encoding the guide RNA may be delivered to a cell by hydrodynamic needle.

Thus, the invention provides the protected DNA product described herein for use in CRISPR system delivery to a cell. The invention provides a method of delivering the CRISPR system to a cell, comprising contacting the protected DNA product described herein with a cell. The invention also provides the protected DNA product obtainable by the methods described herein for use in CRISPR system delivery to a cell. The invention provides a method of delivering the CRISPR system to a cell, comprising contacting the protected DNA product obtainable by the methods described herein with a cell.

The cell may be an animal cell, preferably mammal cell, such as human cell.

The invention also provides a cell obtainable by the methods described herein. The cell is particularly suitable for use in cell therapy and/or in vivo therapy.

The protected DNA product produced or obtainable by the methods described herein may be used in transcription, to generate RNA, preferably mRNA, in vitro or in vivo.

### 7. Kits

The invention provides a kit comprising components required to carry out the method described herein.

The kit may comprise:
(a) an endonuclease;
(b) a polymerase; and
(c) nuclease-resistant deoxynucleotide triphosphates.

The kit may further comprise an exonuclease.

The kit may further comprise at least one buffer.

Each aspect or embodiment as defined herein may be combined with any other aspect(s) or embodiment(s) unless clearly indicated to the contrary. In particular, any feature indicated as being preferred or advantageous may be combined with any other feature or features indicated as being preferred or advantageous.

The foregoing detailed description has been provided by way of explanation and illustration, and is not intended to limit the scope of the appended claims. Many variations in the presently preferred embodiments illustrated herein will be apparent to one of ordinary skill in the art, and remain within the scope of the appended claims and their equivalents.

### BRIEF DESCRIPTION OF THE DRAWINGS

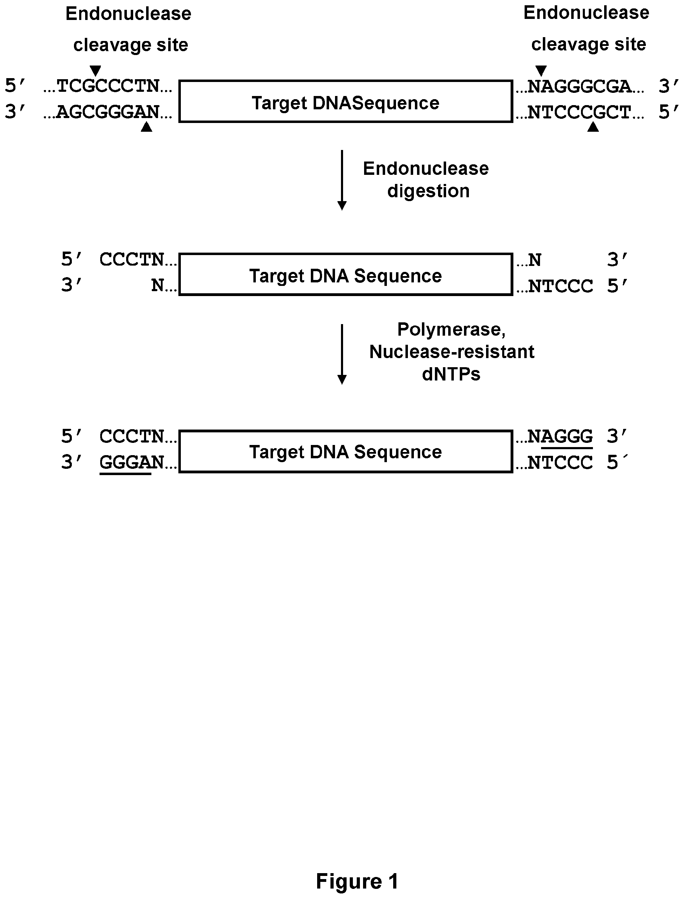

**FIG.** 1 illustrates an exemplary workflow to obtain a protected DNA product by endonuclease digestion followed by extension using a polymerase. Endonuclease digestion produces protruding 5' ends at both sides of a target DNA sequence (i.e. sequence of interest). A polymerase, in the presence of nuclease-resistant deoxynucleotide triphosphates, extends the 3' ends resulting in a protected DNA product due to the presence of nuclease-resistant nucleotides (underlined) at both sides and in both strands, which prevents 3' to 5' exonuclease degradation of the protected DNA product.

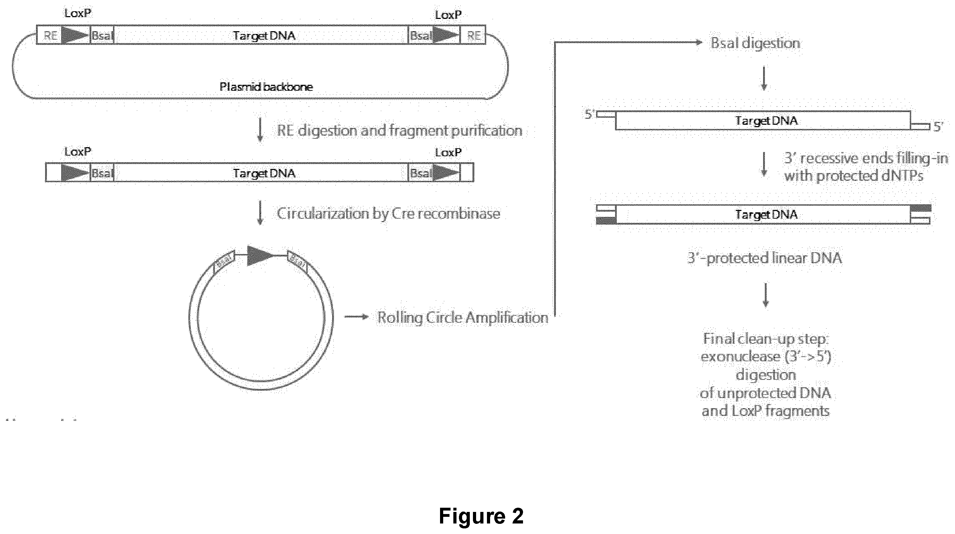

**FIG. 2** illustrates a workflow to obtain 3'-protected linear DNA product by digestion and extension of 3'-recessive ends with phosphorothioated dNTPs, starting from amplified DNA obtained through rolling circle amplification (RCA) of a circular DNA template generated through the action of Cre recombinase on substrates containing two LoxP sequences (SEQ ID NO: 1) flanking the DNA of interest in the same direction.

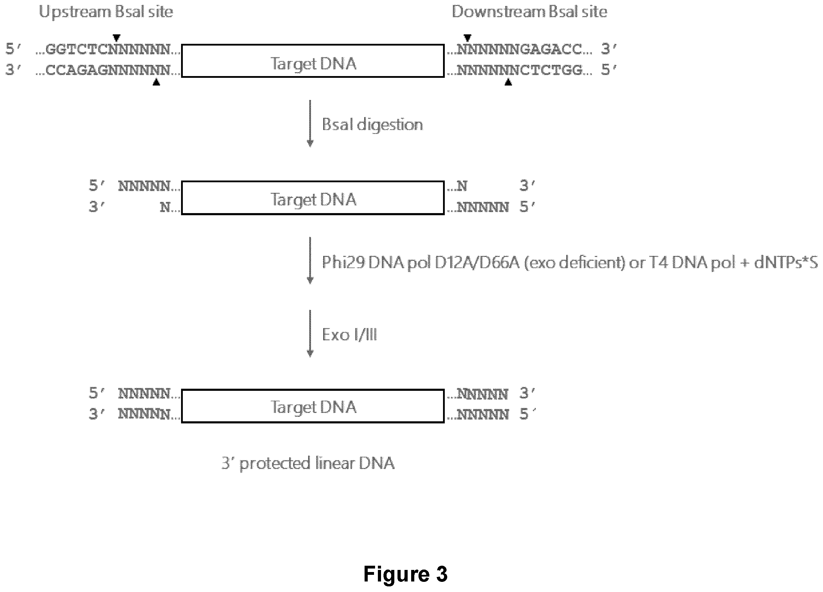

**FIG. 3** illustrates the production of a protected DNA product by Bsal digestion of an amplified double-stranded DNA molecule. Bsal digestion produces 4-nucleotide protruding 5' ends at both sides of the expression cassette. Phosphorothioated dNTPs are then incorporated at 3' recessive ends at both sides of the expression cassette by an exonuclease-deficient DNA polymerase (e.g. T4 DNA polymerase or Phi29 DNA polymerase D12A/D66A double mutant), resulting in 3'-protected DNA product.

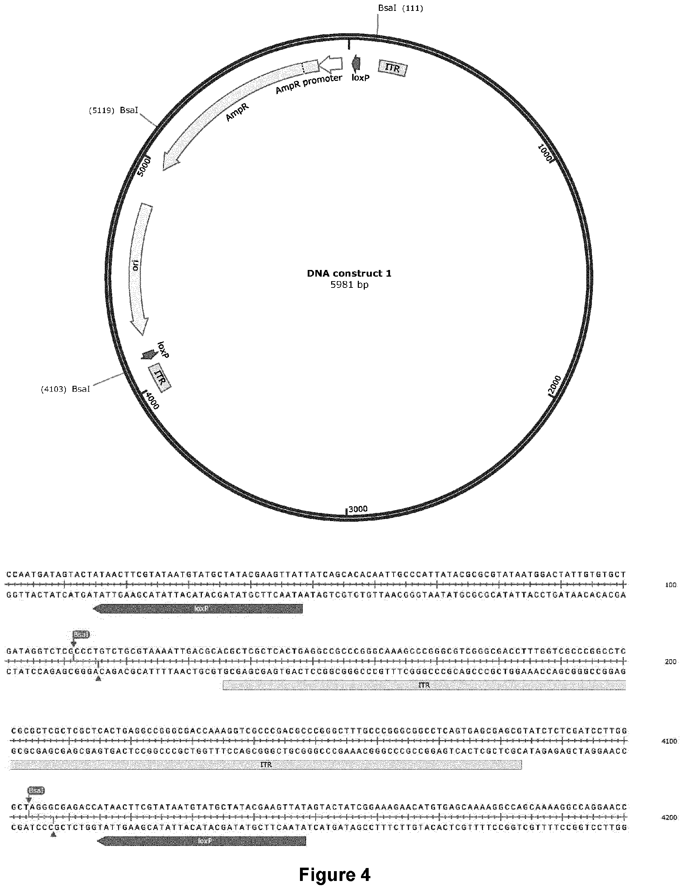

**FIG. 4** illustrates the sequence elements of DNA construct 1. The DNA sequence of interest is flanked by two LoxP sequences (SEQ ID NO: 1) having the same orientation and two inverted terminal repeats (ITRs, SEQ ID NO: 2), required for adeno-associated virus (AAV) replication and encapsidation of the DNA into viral particles. Bsal digestion sites are shown to illustrate the 4-nucleotide protruding ends (TCCC 5') generated at both sides of the expression cassette.
**FIG. 5** depicts the analysis of the amplified/purified DNAs (construct 1) digested with Bsal and extended by Phi29 DNA pol D12A/D66A double mutant (1x) using phosphorothioated dATP and dGTP (10 µM) by agarose gel electrophoresis (0.8%).
**FIG. 6** depicts the analysis of the amplified/purified DNAs (construct 1) digested with Bsal and extended by Phi29 DNA pol D12A/D66A double mutant (1x) using phosphorothioated dATP and dGTP (100 µM and 1 Mm) by agarose gel electrophoresis (0.8%).
**FIG. 7** depicts the analysis of the amplified/purified DNAs (construct 1) digested with Bsal and extended by 10-fold less Phi29 DNA pol D12A/D66A double mutant (0.1×) using phosphorothioated dATP and dGTP (10 µM) by agarose gel electrophoresis (0.8%).
**FIG. 8** depicts the analysis of the amplified/purified DNAs (construct 1) digested with Bsal and extended by 10-fold less Phi29 DNA pol D12A/D66A double mutant (0.1×) using phosphorothioated dATP and dGTP (100 µM and 1 mM) by agarose gel electrophoresis (0.8%).
**FIG. 9** depicts the analysis of the amplified/purified DNAs (construct 1) digested with Bsal and extended by Phi29 DNA pol D12A/D66A double mutant (1x) using phosphorothioated dATP and dGTP (1 mM) by agarose gel electrophoresis (0.8%). The effect of two consecutive exonuclease treatments is shown.
**FIG. 10** depicts the analysis of the amplified/purified DNAs (construct 1) digested with Bsal and extended by 10-fold less Phi29 DNA pol D12A/D66A double mutant (0.1×) using phosphorothioated dATP and dGTP (1 mM) by agarose gel electrophoresis (0.8%). The effect of two consecutive exonuclease treatments is shown.
**FIG. 11** depicts the analysis of the amplified/purified DNAs (construct 1) digested with Bsal and extended by 10-fold less Phi29 DNA pol D12A/D66A double mutant (0.1×) using only phosphorothioated dATP (1 mM) by agarose gel electrophoresis (0.8%). The effect of two consecutive exonuclease treatments is shown.
**FIG. 12** depicts the absence of protection against 5' -> 3' exonucleases of the 3'-protected DNAs (construct 1) generated in Figures 5 and 6 by agarose gel electrophoresis (0.8%).
**FIG. 13** depicts the absence of protection against 5' -> 3' exonucleases of the 3'-protected DNAs (construct 1) generated in Figures 7 and 8 by agarose gel electrophoresis (0.8%).
**FIG. 14** depicts the analysis of the amplified/unpurified DNA (construct 1) digested with Bsal and extended by Phi29 DNA pol D12A/D66A double mutant (0.1× and 1×) using phosphorothioated dATP and dGTP (1 mM of each) by agarose gel electrophoresis (0.8%).
**FIG. 15** depicts the analysis of the amplified/unpurified DNA (construct 1) simultaneously digested with Bsal and extended by Phi29 DNA pol D12A/D66A double mutant (0.1× and 1×) using phosphorothioated dATP and dGTP (1 mM of each) by agarose gel electrophoresis (0.8%). The effect of two consecutive exonuclease treatments is shown.
**FIG. 16** depicts the analysis of the amplified/unpurified DNA (construct 1) simultaneously digested with Bsal and extended by Phi29 DNA pol wild-type (WT) (0.1× and 1x) or D12A/D66A double mutant (0.1×) using phosphorothioated dATP and dGTP (1 mM of each) by agarose gel electrophoresis (0.8%).
The effect of two consecutive exonuclease treatments is shown.
**FIG. 17** depicts the analysis of the 3'-protected DNAs generated by DNA digestion and 3'-protection carried out simultaneously using Klenow Fragment.

The sequences discussed in the application are provided in the table below:

**Table 1. Sequences discussed in the application.**

| **SEQ ID NO** | **Sequence** |
|---|---|
| SEQ ID NO: 1 | ATAACTTCGTATAATGTATGCTATACGAAGTTAT |
| SEQ ID NO: 2 | CGCTCGCTCACTGAGGCCGCCCGGGCAAAGCCCGGGCGTCGGGCGAC CTTTGGTCGCCCGGCCTCAGTGAGCGAGCGAGCGCGCAGAGAGGGAGT GGCCAACTCCATCACTAGGGGTTCCT |

### EXAMPLES

### Example 1: Sequential generation of 3'-protected DNA

Cre recombinase from the P1 bacteriophage is a Type I topoisomerase. The enzyme catalyzes the site-specific recombination of DNA between loxP sites (SEQ ID NO: 1). LoxP recognition site (34 bp) consists of two 13 bp inverted repeats which flank an 8 bp spacer region, which confers directionality. The products of Cre-mediated recombination are dependent upon the location and relative orientation of the IoxP sites. Two DNA species containing single IoxP sites were fused. DNA found between two IoxP sites oriented in the same direction was excised as a circular loop of DNA, while DNA between opposing IoxP sites was inverted with respect to external sequences. Cre recombinase requires no additional cofactors or accessory proteins for its function.

Cre reaction conditions: reaction volume 500 µl, DNA of interest purified from agarose gel electrophoresis after restriction enzyme digestion (1 µg), Cre recombinase (NEB, 40 units), incubation time and temperature: 30 min at 37°C and 20 min at 80°C. Next, to remove remaining non-circular DNA molecules before the amplification step, *E.* coli exonuclease I (NEB, 200 units) and III (NEB, 1000 units) were added and the reaction was incubated 30 min at 37°C and 20 min at 80°C.

Rolling circle amplification (RCA) is a faithful and isothermal DNA amplification method based on Phi29 DNA polymerase (Phi29DNApol). Phi29DNApol is the monomeric enzyme responsible for the replication of the linear double stranded DNA of bacteriophage phi29 from *Bacillus subtilis* (Blanco and Salas, Proc Natl Acad Sci USA. 1984 Sep;81(17):5325-9). It is an extremely processive polymerase (up to more than 70 kb per binding event) with a strong strand displacement capacity (Blanco et al., J Biol Chem. 1989 May 25;264(15):8935-40). The enzyme displays 3'->5' proofreading exonuclease activity (Garmendia et al., J Biol Chem. 1992 Feb 5;267(4):2594-9), resulting in an extremely high fidelity of synthesis (Esteban et al., J Biol Chem. 1993 Feb 5;268(4):2719-26 . These special features make this enzyme the perfect choice for isothermal DNA amplification.

RCA can be initiated by random synthetic primers (Dean et al., Genome Res. 2001 Jun;11(6):1095-9) or a DNA primase like *Tth*PrimPol (Picher et al., Nat Commun. 2016 Nov 29;7:13296 ) that synthesizes the primers for Phi29DNApol during the amplification reaction.

Before the amplification, circularized DNA samples were first denatured by adding 1 volume of buffer D (400 mM KOH, 10 mM EDTA) and incubating 3 min at room temperature. Samples were then neutralized by adding 1 volume of buffer N (400 mM HCl, 600 mM Tris-HCl pH 7.5). Rolling circle amplification conditions: 10 ml reaction volume, 1 ml TruePrime WGA reaction buffer 10x (4basebio), 500 µl denatured DNA sample, 1 ml *Tth*PrimPol (1 µM), 160 µl QualiPhi Phi29DNApol (12,5 µM), 2.5 units PPase (Thermo) and 1 ml dNTPs (10 mM). Incubation time and temperature: 20 hours at 30°C and 10 min at 65°C.

Amplified DNA was then processed in different ways. An amplified DNA fraction was first digested with Bsal (DNA = 240 ng/µl; Bsal-HF (NEB) = 0,15 U/µl; 23 hours at 30ºC) and then purified by size exclusion using Sephadex G-25 or G-50 to remove remaining dNTPs from the amplification step, resulting in DNA substrates named "DNA 1" and "DNA 2" respectively. Another amplified DNA fraction was first ethanol-precipitated to remove remaining dNTPs from the amplification step before being digested with Bsal (DNA = 240 ng/µl; Bsal-HF (NEB) = 0,15 U/µl; 23 hours at 30ºC), resulting in the sample named "DNA 3". Finally, the last amplified DNA fraction was first purified by size exclusion using Sephadex G-25 or G-50, and then digested with Bsal (DNA = 240 ng/µl; Bsal-HF (NEB) = 0,15 U/µl; 23 hours at 30ºC), resulting in substrates named "DNA 4" and "DNA 5" respectively.

Digested/purified DNAs 1-5 were then incubated with Phi29 DNA polymerase D12A/D66A double mutant (exonuclease deficient) and complementary phosphorothioated nucleotides to the 5' protruding ends generated by Bsal. Filling-in (polymerase extension) reaction conditions: TruePrime RB 1x, DNA = 120 ng/µl, 1,3 (0.1×) or 13 (1×) ng/µl Phi29 DNA pol D12A/D66A, increasing concentrations of dATP*S and dGTP*S, 20 hours at 30ºC and 10 minutes at 65ºC.

After the reaction to incorporate protected dNTPs into 3' recessive ends, exonucleases I and III were added to remove single- and double-stranded unprotected DNA molecules remaining. Exonuclease clean-up reaction conditions: 1,1 ng/µl of *E. coli*exonuclease I and 1,9 ng/µl of *E. coli*exonuclease III. Incubation time and temperature: 2 hours at 37°C.

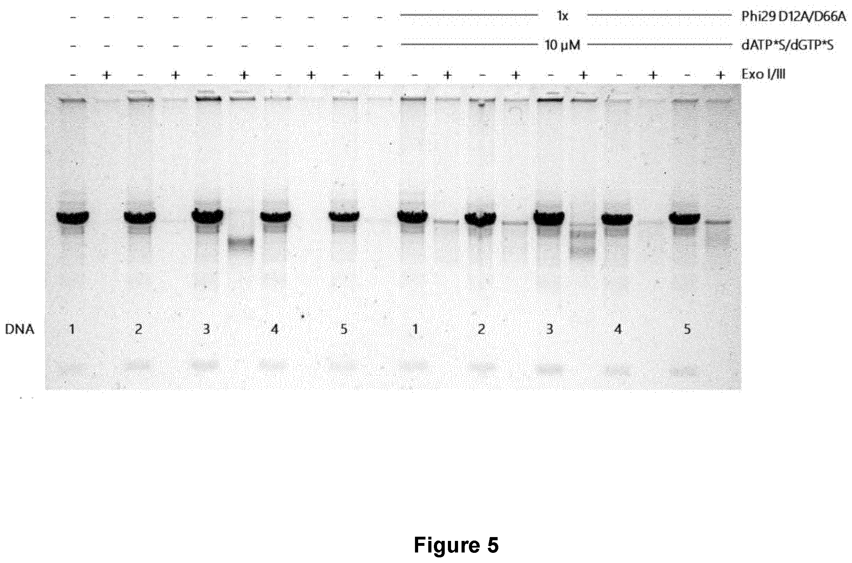

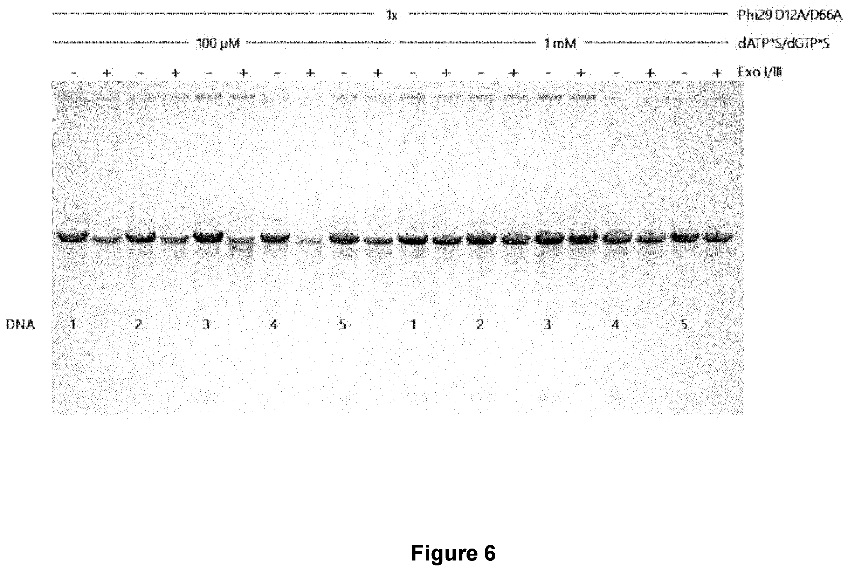

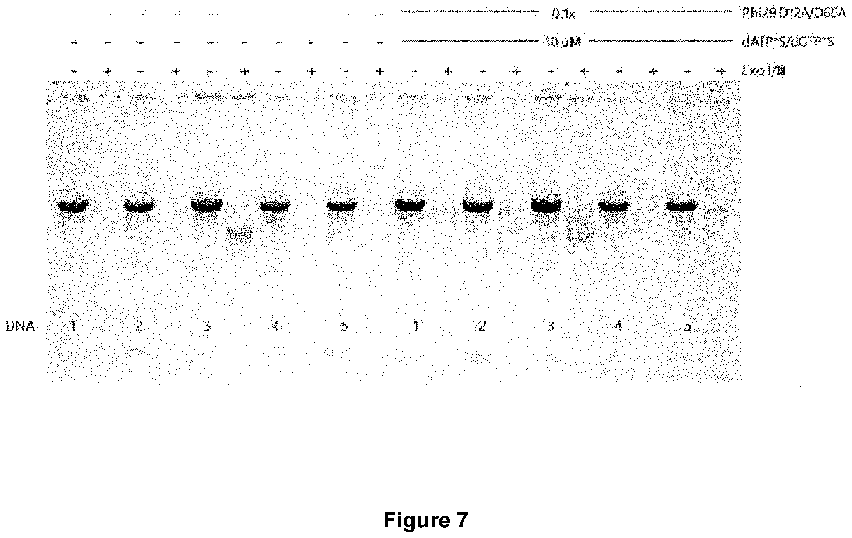

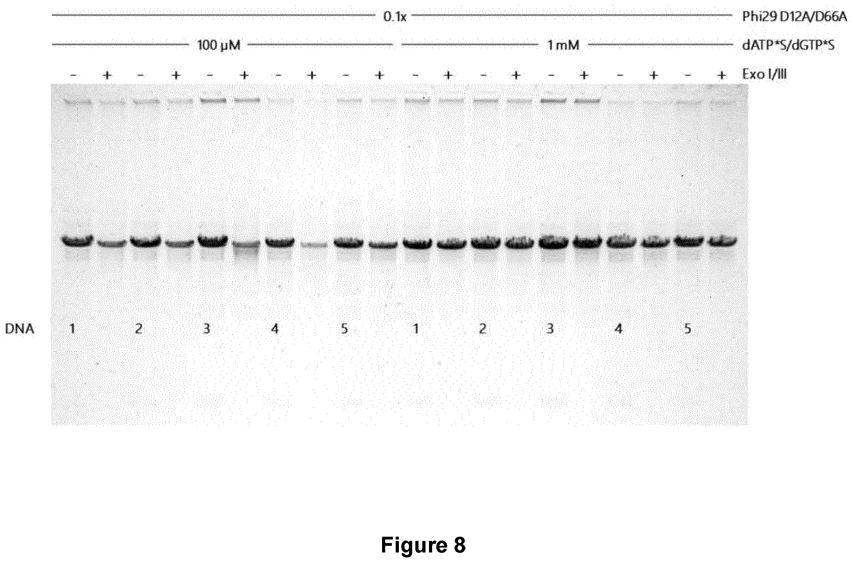

Shown in FIG. 5, 6, 7 and 8 are the agarose gel electrophoresis analysis of the digested/purified DNAs 1-5 processed by two different doses of Phi29 DNA polymerase D12A/D66A with increasing amounts of the complementary phosphorothioated nucleotides to the 5' protruding ends previously generated by Bsal. The absence of both components (polymerase and nucleotides) resulted in almost complete degradation of the starting DNA material (See FIG. 5 and 7). However, increasing concentrations of complementary phosphorothioated nucleotides in the presence of Phi29 DNA polymerase D12A/D66A resulted in the generation of DNA material resistant to exonucleolytic degradation.

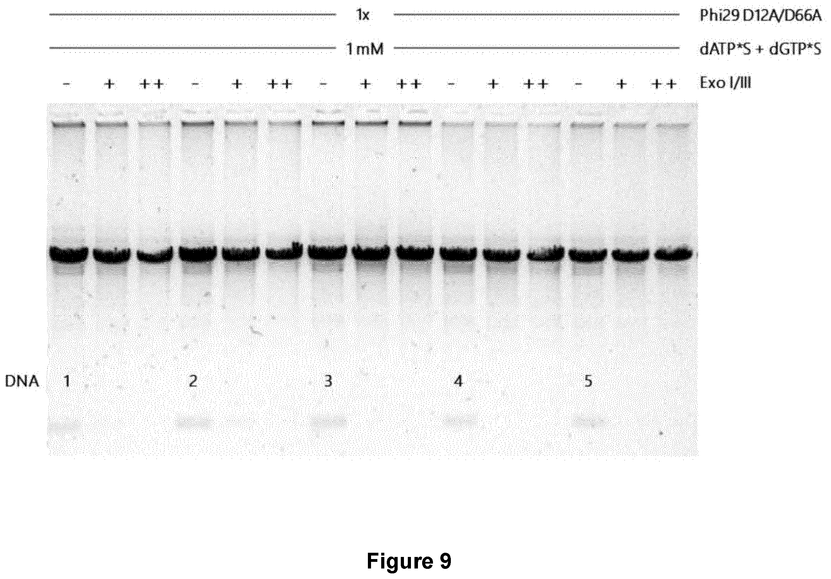

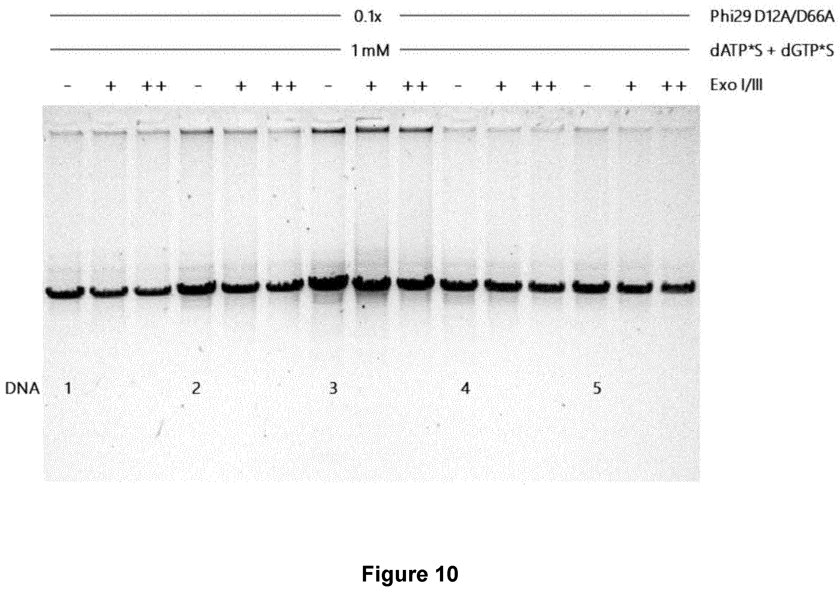

Shown in FIG. 9 and 10 are the agarose gel electrophoresis analysis of the exonuclease resistant DNAs generated with dATP*S/dGTP*S (1 mM of each) and both doses of Phi29 DNA polymerase D12A/D66A treated twice with *E. coli* exonuclease I and III. The second incubation with the exonucleolytic enzymes did not result in any significant decrease in the amount of resistant DNA remaining.

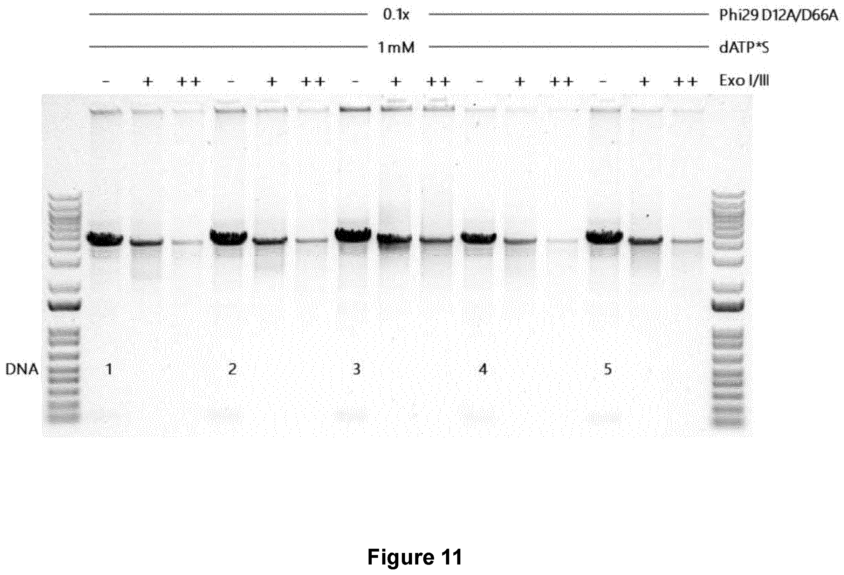

Shown in FIG. 11 is the agarose gel electrophoresis analysis of the digested/purified DNAs 1-5 processed by Phi29 DNA polymerase D12A/D66A in the presence of only one protected dNTP (dATP*S). As opposed to the results observed in FIG. 9 and 10, the amount of material remaining after each exonucleolytic treatment with *E. coli* exonuclease I and III was significantly lower compared to the starting material, suggesting that the use of protected dNTP confers less resistance to exonucleolytic degradation.

3'-protected DNAs obtained with 1x Phi29 DNA pol D12A/D66A and 1 mM dATP*S/dGTP*S after removal of unprotected molecules by *E. coli* exonuclease I and III were then treated with exonucleases acting on double-stranded from 5' to 3' (i.e. T5 exonuclease, T7 exonuclease, *E. coli*exonuclease VIII) to show the absence of protection on 5' ends. Reaction conditions: TruePrime RB 1x, DNA = 120 ng/µl, 10 units of each exonuclease (NEB), 30 minutes at 37ºC (T5 and Exo VIII) or 25ºC (T7).

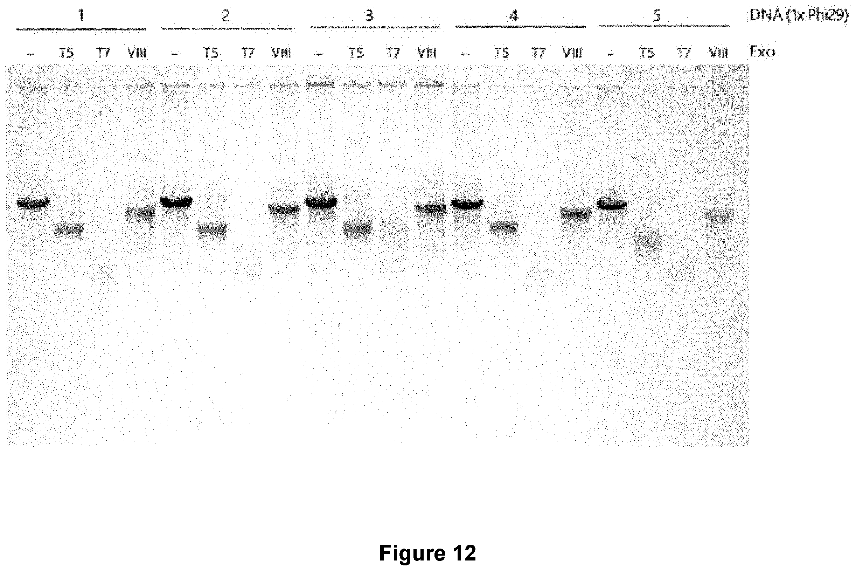

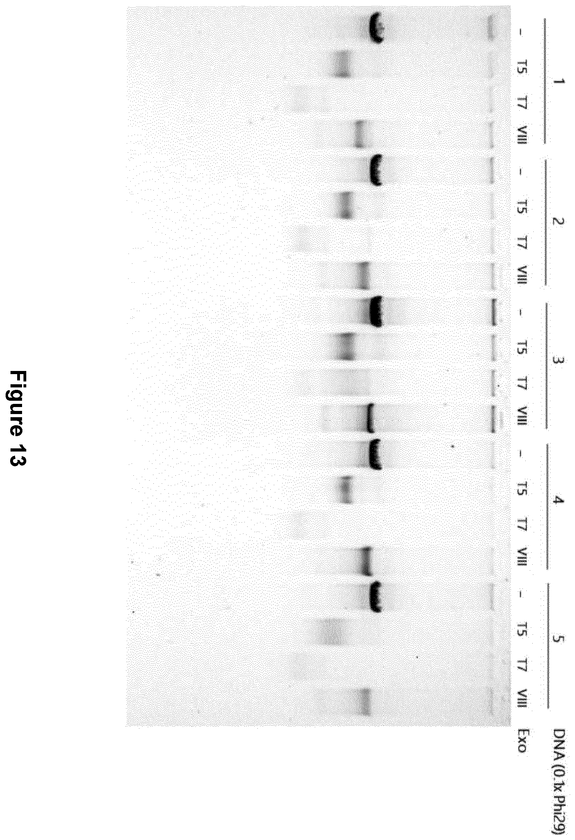

Shown in FIG. 12 and 13 are the agarose gel electrophoresis analysis of the 3'-protected DNAs generated with dATP*S/dGTP*S (1 mM of each) and both doses of Phi29 DNA polymerase D12A/D66A treated with T5 exonuclease, T7 exonuclease or *E. coli* exonuclease VIII. As expected, none of the DNAs showed resistance to degradation by any of the exonucleases tested.

### Example 2: Amplified DNA purification is not required to protect 3' ends

Amplified DNA was digested with Bsal (DNA = 240 ng/µl; Bsal-HF (NEB) = 0,15 U/µl; 23 hours at 30ºC) and then incubated with Phi29 DNA polymerase D12A/D66A and the complementary phosphorothioated nucleotides to the 5' protruding ends generated by Bsal. No purification step was carried out before or after Bsal digestion. Extension reaction conditions: TruePrime RB 1x, DNA = 120 ng/µl, 1,3 (0.1×) or 13 (1x) ng/µl Phi29 DNA pol D12A/D66A, dATP*S/dGTP*S (1 mM of each), 20 hours at 30ºC and 10 minutes at 65ºC. After the reaction to incorporate protected dNTPs into 3' recessive ends, exonucleases I and III were added to remove single- and double-stranded unprotected DNA molecules remaining. Exonuclease clean-up reaction conditions: 1,1 ng/µl of *E. coli* exonuclease I and 1,9 ng/µl of *E. coli* exonuclease III. Incubation time and temperature: 2 hours at 37°C.

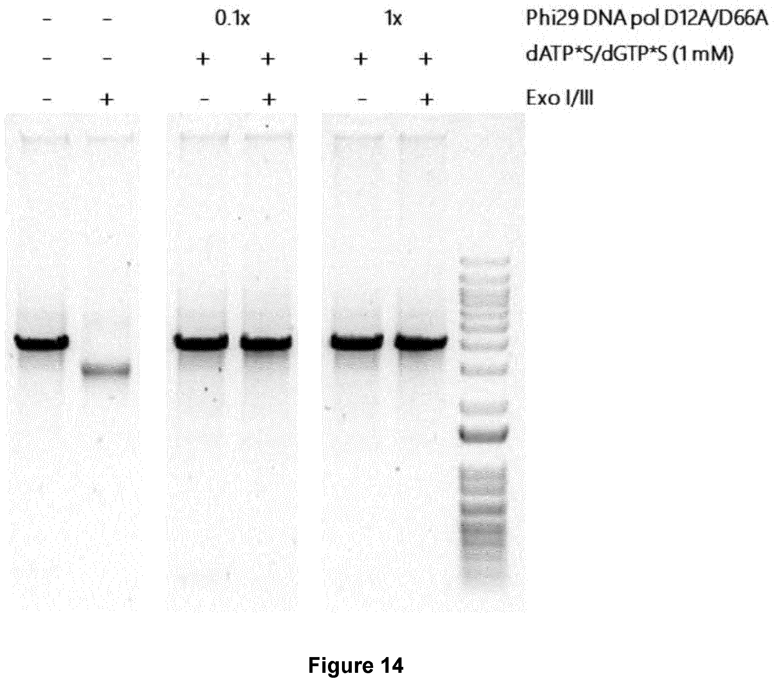

Shown in FIG. 14 is the agarose gel electrophoresis analysis of the 3'-protected DNAs generated with dATP*S/dGTP*S (1 mM of each) and both doses of Phi29 DNA polymerase D12A/D66A starting from unpurified amplified DNA digested with Bsal. The efficiency of the 3'-protection procedure is like those obtained when purifying the material before the reaction with Phi29 DNA polymerase D12A/D66A and protected dNTPs. Therefore, this data shows that protected DNA products can be generated without a purification step between DNA amplification and endonuclease digestion.

### Example 3: Amplified DNA digestion and 3'-protection can be carried out simultaneously using Phi29

Amplified DNA was incubated with Bsal, Phi29 DNA polymerase D12A/D66A and the complementary phosphorothioated nucleotides to the 5' protruding ends generated by Bsal to check if digestion and protection steps could be performed simultaneously. Reaction conditions: TruePrime RB 1x, DNA = 120 ng/µl, 0,15 U/µl Bsal, 1,3 (0.1×) or 13 (1x) ng/µl Phi29 DNA pol D12A/D66A, dATP*S/dGTP*S (1 mM of each), 23 hours at 30ºC and 10 minutes at 65ºC. After the reaction, exonucleases I and III were added to remove single- and double-stranded unprotected DNA molecules remaining. Exonuclease clean-up reaction conditions: 1,1 ng/µl of *E. coli*exonuclease I and 1,9 ng/µl of *E*. *coli*exonuclease III. Incubation time and temperature: 2 hours at 37°C. The exonuclease treatment was performed twice consecutively.

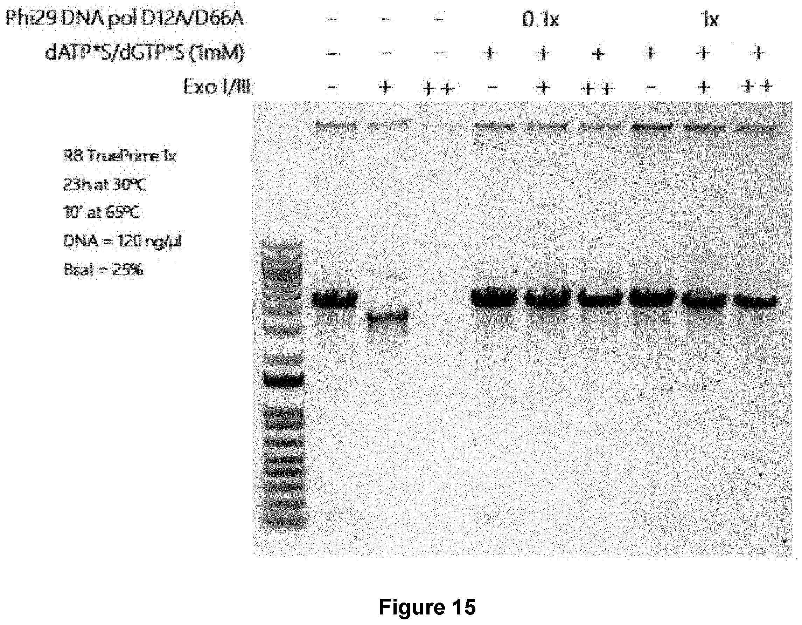

Shown in FIG. 15 is the agarose gel electrophoresis analysis of the 3'-protected DNAs generated with the simultaneous incubation of unpurified amplified DNA with Bsal, Phi29 DNA polymerase D12A/D66A and the complementary phosphorothioated nucleotides. The efficiency of the 3'-protection procedure is similar to when purifying the material before the reaction with Phi29 DNA polymerase D12A/D66A and protected dNTPs.

### Example 4: Exonuclease proficient Phi29 DNA polymerase can also efficiently protect 3' ends

Amplified DNA was incubated with Bsal, Phi29 DNA polymerase wild-type and the complementary phosphorothioated nucleotides to the 5' protruding ends generated by Bsal to check if the 3'-5' exonuclease activity of Phi29 DNA polymerase could prevent the protection by removing the incorporated phosphorothioated nucleotides. Reaction conditions: TruePrime RB 1x, DNA = 120 ng/µl, 0,15 U/µl Bsal, 1,3 (0.1×) or 13 (1x) ng/µl Phi29 DNA pol, dATP*S/dGTP*S (1 mM of each), 23 hours at 30ºC and 10 minutes at 65ºC. After the reaction, exonucleases I and III were added to remove single- and double-stranded unprotected DNA molecules remaining. Exonuclease clean-up reaction conditions: 1,1 ng/µl of *E. coli* exonuclease I and 1,9 ng/µl of *E. coli* exonuclease III. Incubation time and temperature: 2 hours at 37°C. The exonuclease treatment was performed twice consecutively.

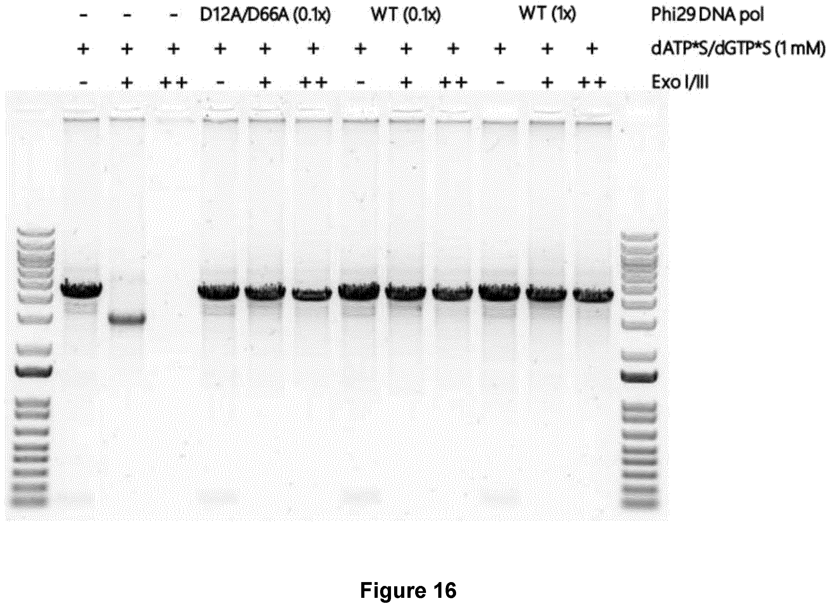

Shown in FIG. 16 is the agarose gel electrophoresis analysis of the 3'-protected DNAs generated with the simultaneous incubation of unpurified amplified DNA with Bsal, Phi29 DNA polymerase wild-type or D12A/D66A and the complementary phosphorothioated nucleotides. No significant difference in the efficiency of the 3'-protection procedure is observed when comparing wild-type Phi29 DNA polymerase (exonuclease proficient) and the exonuclease deficient version (D12A/D66A), independently of Phi29 DNA pol wild-type concentration.

### Example 5: Amplified DNA digestion and 3'-protection can be carried out simultaneously using Klenow Fragment

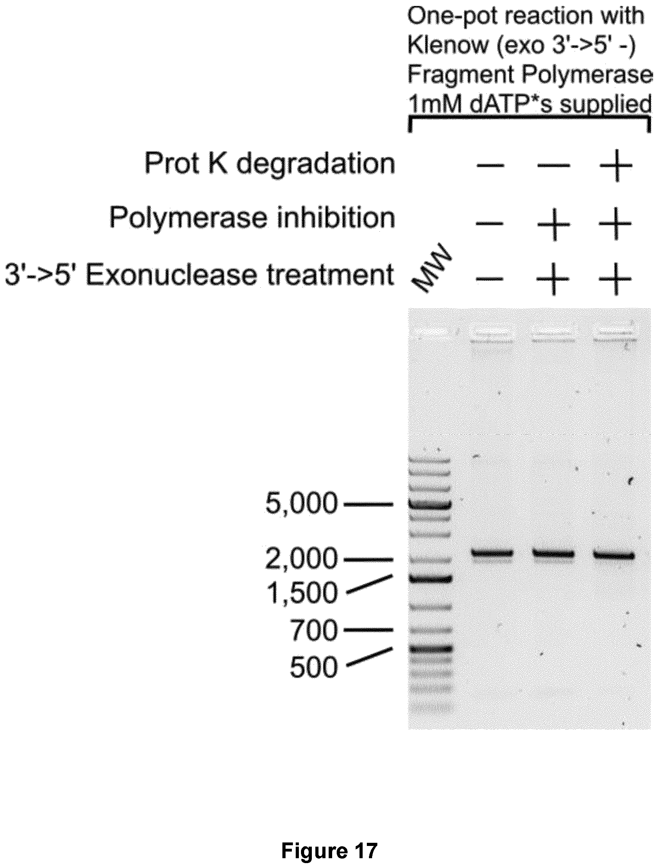

Amplified DNA was incubated with Bsal, Klenow Fragment (3'→5' exo-) (NEB) and the complementary phosphorothioated nucleotides to the 5' protruding ends generated by Bsal. Reaction conditions: amplified DNA at 120 ng/uL was incubated with TruePrime RB 1x, 0,15 U/µl Bsal, 0.07 U/uL Klenow Fragment (3'→5' exo-) (NEB), 1 mM dATP*S, incubated for 20 hours at 30°C and inactivated for 10 minutes at 65°C (see "Polymerase inhibition" in FIG. 17), followed by 1,1 ng/µl of E. coli exonuclease I and 1,9 ng/µl of E. coli exonuclease III (see "3'→5' Exonuclease treatment" in FIG. 17).

Shown in FIG. 17 is the agarose gel electrophoresis analysis of the 3'-protected DNAs generated by this experiment. This shows that the Klenow Fragment (3'→5' exo-) (NEB) successfully incorporated protected dATP*S onto the 3' ends of the linear DNA generated by Bsal. The Polymerase inhibition (i.e. heat inactivation) and Proteinase K treatments demonstrate that DNA is protected solely via dATP* incorporation and not any enzyme intervention present during the exonuclease treatment.

In the Examples, dATP*S is α-S-dATP (i.e. 2'-deoxyadenosine-5'-(α-thio)-triphosphate) and dGTP*S is α-S-dGTP (i.e. 2'-deoxyguanosine-5'-(α-thio)-triphosphate).

## Claims

1. A method for producing a protected deoxyribonucleic acid (DNA) product, wherein the method comprises:
(a) amplifying a DNA template molecule to generate a double-stranded DNA molecule, wherein the amplifying comprises rolling circle amplification;
(b) digesting the double-stranded DNA molecule with an endonuclease to generate a digested double-stranded DNA molecule comprising a first strand and a second strand, wherein the first strand has a 5' overhang; and
(c) extending the second strand 5' to 3' using a polymerase in the presence of one or more nuclease-resistant deoxynucleotide triphosphates to generate the protected DNA product, wherein the protected DNA product comprises an extended second strand comprising at least one nuclease-resistant nucleotide incorporated into the extended second strand by the polymerase using the 5' overhang of the first strand as template.

2. The method of claim 1, wherein in step (b) the first strand has a 5' overhang and the second strand has a 5' overhang and wherein step (c) comprises:
extending the first strand and the second strand 5' to 3' using a polymerase in the presence of one or more nuclease-resistant deoxynucleotide triphosphates to generate the protected DNA product, wherein the protected DNA product comprises (i) an extended first strand comprising at least one nuclease-resistant nucleotide incorporated into the extended first strand by the polymerase using the 5' overhang of the second strand as template, and (ii) an extended second strand comprising at least one nuclease-resistant nucleotide incorporated into the extended second strand by the polymerase using the 5' overhang of the first strand as template.

3. The method of claim 1 or claim 2, wherein the 5' overhang is at least 4 nucleotides in length.

4. The method of any one of claims 1 to 3, wherein the 5' overhang consists of one, two, three or four types of nucleotide selected from cytosine, guanine, thymine and/or adenine.

5. The method of any one of claims 1 to 4, wherein the nuclease-resistant deoxynucleotide triphosphates consist of one, two, three or four types of nuclease-resistant deoxynucleotide triphosphate selected from guanine triphosphate, cytosine triphosphate, adenine triphosphate and/or thymine triphosphate.

6. The method of any one of claims 1 to 5, wherein the endonuclease is a restriction endonuclease or RNA-guided DNA endonuclease.

7. The method of any one of claims 1 to 6, wherein steps (b) and (c) are performed in a single contiguous volume.

8. The method of any one of claims 1 to 7, wherein the nuclease-resistant deoxynucleotide triphosphates and the nuclease-resistant nucleotides are phosphorothioated.

9. The method of any one of claims 1 to 8, wherein the polymerase lacks exonuclease activity and/or strand displacement activity.

10. The method of any one of claims 1 to 9, wherein the amplification in step (a) is performed in the absence of nuclease-resistant deoxynucleotide triphosphates.

11. The method of any one of claims 1 to 10, wherein no purification step is performed after the amplification in step (a) and before steps (b) and (c).

12. The method of any one of claims 1 to 11, wherein (i) the extended first strand comprises at least two nuclease-resistant nucleotides incorporated into the extended first strand by the polymerase using the 5' overhang of the second strand as template, and/or (ii) the extended second strand comprises at least two nuclease-resistant nucleotides incorporated into the extended second strand by the polymerase using the 5' overhang of the first strand as template.

13. The method of any one of claims 1 to 12, wherein the protected DNA product is linear.

14. A method for *in vitro* transcription of a protected DNA product, wherein the method comprises:
(a) producing a protected DNA product according to the method of any one of claims 1-13;
(b) contacting the protected DNA product with a polymerase; and
(c) producing a transcription product encoded by the protected DNA product.

15. Use of a protected DNA product in the production of a viral or non-viral delivery system, wherein the protected DNA product is produced by the method of any one of claims 1-13.
